# EUROPEAN PATENT APPLICATION

(11) **EP 4 834 919 A1**
(43) Date of publication of application: **04.11.2026**
(21) Application number: 24910458.9
(22) Date of filing: 25.11.2024
(51) Int. Cl.: C07K 14/47, C07K 19/00, C12N 15/70, A61K 39/35, A61P 37/08, G01N 33/00

(54) **RECOMBINANT CAT ALLERGEN, PHARMACEUTICAL COMPOSITION THEREOF, PREPARATION METHOD THEREFOR, AND USE THEREOF**

(30) Priority: 28.12.2023 CN 202311827404; 28.12.2023 CN 202311827552; 28.12.2023 CN 202311827407
(71) Applicant: Zonhon Biopharma Institute, Inc., Changzhou, Jiangsu 213125 (CN)
(72) Inventor: MA, Bruce Yong, Changzhou, Jiangsu 213125 (CN); ZHAO, Baixue, Changzhou, Jiangsu 213125 (CN); GAO, Hui, Changzhou, Jiangsu 213125 (CN); WU, Cheng, Changzhou, Jiangsu 213125 (CN)
(74) Representative: Lorenz Seidler Gossel Part. mbB
(86) International application number: PCT/CN2024/134148
(87) International publication number: WO 2025/139524

(57) **Abstract**

Provided are recombinant cat allergens, pharmaceutical compositions thereof, methods for preparation and applications thereof. Compared with natural allergen extract products, the recombinant cat allergens possess numerous advantages for allergen-specific immunotherapy and the diagnosis of cat allergic diseases. These advantages include high purity and high homogeneity; the provision of pure proteins/peptides with defined properties and quality; predetermined allergenicity, immunogenicity, and tolerability; manufacturing in accordance with Good Manufacturing Practice (GMP) standards; and reproducible production at defined quantities and concentrations, thereby enabling product standardization and robust quality control in compliance with modern regulatory requirements for pharmaceuticals and vaccines. In diagnostic applications, the recombinant cat allergens can identify the specific allergen molecules responsible for allergic reactions and reveal cross-reactivity; in therapeutic applications, they allow targeted allergen immunotherapy, avoiding interference from unknown non-allergenic substances in extracts, improving safety, and facilitating precise diagnosis and precise administration.

## Description

### TECHNICAL FIELD

The present invention belongs to the field of biopharmaceutical technology, and relates to recombinant cat allergens, as well as the methods for preparation and the use thereof.

### BACKGROUND

Allergic diseases, also known as atopic diseases, are primarily type I hypersensitivity reactions mediated by immunoglobulin E (IgE). The occurrence of allergy can be divided into two stages: sensitization and elicitation (the allergic phase). During the sensitization stage, upon first exposure of the body to an allergen, B cells are stimulated to produce allergen-specific IgE antibodies; during this period, the body exhibits no abnormal response. During the elicitation stage, when the same allergen re-enters the sensitized body once or multiple times, IgE antibodies on the surface of mast cells or basophils bind to and are cross-linked by the allergen, resulting in degranulation of mast cells and basophils and the release of a series of biologically active mediators, thereby inducing allergic reactions (also referred to as hypersensitivity reactions, allergy) and related symptoms, such as allergic asthma, hay fever, urticaria, allergic rhinitis, eczema, conjunctivitis, gastrointestinal type I allergic diseases, and severe anaphylactic reactions. The incidence of allergic diseases has been increasing year by year and has become a global public health concern. The World Health Organization (WHO) has listed allergic diseases as a key focus for research, prevention and treatment in the 21st century.

Cat allergens are among the most important indoor allergens and are a common cause of allergic rhinitis (AR) and bronchial asthma (BA). Studies have shown that approximately 30% of patients with AR and BA are allergic to cats, and that the more frequently children under two years of age are exposed to cat allergens, the more likely they are to develop cat-related allergic diseases in adulthood.

Current therapies for allergic diseases caused by cat allergens include specific immunotherapy (SIT) and anti-allergic therapy. Specific immunotherapy, also known as allergen immunotherapy, involves administering gradually increasing doses of an allergen to a patient, via subcutaneous injection or sublingual administration, to repeatedly expose the patient to the allergen and thereby enhance tolerance to the allergen. When the patient is subsequently exposed to the same allergen, the release of inflammatory mediators is significantly reduced, and the severity of specific hypersensitivity reactions is markedly alleviated, thereby relieving clinical symptoms and ultimately achieving tolerance or even immune tolerance. SIT is currently the only etiological treatment that may influence the natural course of allergic diseases and modify the immune response mechanism. In addition to the above therapies, other approaches include avoiding contact with cats, and immunizing cats with their own major allergens (primarily Fel d 1) to induce neutralizing antibodies and thereby reduce endogenous allergen levels.

The active ingredients of specific immunotherapy drugs are major allergenic proteins, which are capable of inducing hypersensitivity reactions. Major allergenic proteins with well-defined compositions can be obtained through allergen extraction or recombinant expression. For example, the Dermatophagoides farinae drops and the Artemisia annua pollen allergen sublingual drops marketed in China by Wolwo Bio, as well as the house Dermatophagoides allergen preparations marketed in Europe by ALK-Abelló, all use natural allergen extracts as active ingredients.

However, natural allergen extracts inevitably present quality-related issues due to limitations in raw material sources and production methods, such as the presence of undefined non-allergenic substances, contaminants, and high variability in allergen content and biological activity (Valenta R, et al., Journal of Allergy & Clinical Immunology in Practice, 2018). The 2018 guidelines on allergen immunotherapy for allergic rhinoconjunctivitis issued by the European Academy of Allergy and Clinical Immunology (EAACI) also point out that mixed allergens have several potential disadvantages, including dilution effects, potential allergen degradation due to enzymatic activity of certain allergens, and difficulty in adequately demonstrating the efficacy of allergen combinations.

Recombinant allergens prepared by genetic engineering techniques can achieve high purity and high homogeneity. While possessing the immunological activity of natural allergens, they effectively overcome the aforementioned drawbacks of natural allergen extracts. For example, recombinant allergens can be produced as pure proteins/peptides with defined properties and quality; their allergenicity, immunogenicity, and tolerability can be predetermined; they can be manufactured in accordance with Good Manufacturing Practice (GMP) standards, allowing reproducible production of defined quantities and concentrations; and they enable product standardization and quality control, meeting modern regulatory requirements for pharmaceuticals and vaccines. In diagnostic applications, recombinant allergens can identify the specific allergen molecules responsible for allergic reactions and reveal cross-reactivity; in therapeutic applications, they allow targeted allergen immunotherapy based on the actual sensitizing proteins, avoiding interference from unknown non-allergenic substances in extracts, improving safety, and facilitating precise diagnosis and precise administration (Valenta R, et al., Journal of Allergy & Clinical Immunology in Practice, 2018). At present, no specific immunotherapy drugs specifically targeting cat allergy have been approved for marketing either domestically or internationally.

### SUMMARY

The applicant aims to provide multiple recombinant cat allergens to obtain highly purified and homogeneous allergen proteins with good biological activity, thereby improving product quality controllability and laying a foundation for the precise diagnosis and treatment of allergic diseases caused by cat allergens, as well as for the standardization of cat allergen products.

A first objective of the present invention is to provide a protein for treating allergic diseases caused by cat allergens, where the protein is a recombinant Fel d 1 protein. The recombinant Fel d 1 protein is a fusion protein including Fel d 1 Chain 1 and Chain 2. Native Chain 1 and Chain 2 contain signal peptides, with GenBank accession numbers AAC37318 and AAC41616, respectively. In specific embodiments, the mature peptides without signal peptides are used, with amino acid sequences as set forth in SEQ ID NO: 5 and SEQ ID NO: 6, respectively.

Natural allergens are generally present in multiple isoallergens or variants (isoforms) due to various influencing factors. Isoallergens typically have similar molecular weights, similar or identical biological functions, and amino acid sequence identities greater than 67%. Each isoallergen may have multiple highly homologous sequences (with identity greater than 90%, usually differing by only a few amino acids), referred to as variants or isoforms (WHO/IUIS Allergen Nomenclature: providing a common language). The amino acid sequences of the above-mentioned Chain 1 and Chain 2 may also be derived from other isoallergens or variants.

The recombinant Fel d 1 protein is preferably designed such that, from the N-terminus to the C-terminus, it includes Fel d 1 Chain 1 followed by Chain 2, or Chain 2 followed by Chain 1. Chain 1 and Chain 2 may be directly fused or linked via a linker peptide. In specific embodiments, the amino acid sequences of Fel d 1-1 and Fel d 1-2 are used as examples to illustrate the two fusion orientations, where Chain 1 and Chain 2 are directly fused. As is well known in the art, in most cases, the start codon in prokaryotes is AUG, encoding N-formylmethionine, while in eukaryotes, the start codon AUG encodes methionine.Therefore, recombinant proteins expressed in prokaryotic systems such as Escherichia coli typically contain an N-terminal formylmethionine in addition to the native amino acid sequence, whereas recombinant proteins expressed in eukaryotic systems such as yeast typically contain an N-terminal methionine. For proteins with signal peptides, the amino acid encoded by the start codon is cleaved together with the signal peptide, resulting in the native protein sequence. In addition, the N-terminal formylmethionine of recombinant proteins may, in some cases, be removed, however, such removal is often incomplete, and the extent of cleavage varies depending on the specific protein. In specific embodiments of the present invention, Fel d 1-1 and Fel d 1-2 are expressed in an Escherichia coli system. Accordingly, the corresponding amino acid sequences (SEQ ID NO: 1 and SEQ ID NO: 2) each include an additional N-terminal formylmethionine. Based on the above common knowledge, the recombinant Fel d 1 protein provided herein may also have an amino acid sequence in which the N-terminal formylmethionine of SEQ ID NO: 1 or SEQ ID NO: 2 is absent or replaced with methionine.

Native Fel d 1 (nFel d 1) is a type I cat allergen and a member of secretoglobin family. It is the major allergenic protein and is primarily present in cat saliva, anal glands, sebaceous glands, and dander. Studies have shown that more than 80-95% of cat-allergic patients have Fel d 1-specific IgE antibodies in their serum. Crossed immunoelectrophoresis has demonstrated that most IgE antibodies against cat allergens in allergic patients are Fel d 1-specific, accounting for 69-90% of the total allergenic activity (Benjamin Bonnet et al., Allergy Asthma and Clinical Immunology, 2018).

Native Fel d 1 includes two chains, Chain 1 and Chain 2, which are expressed from their respective genes and undergoing post-translational modifications, followed by assembly into the complete Fel d 1 protein via intermolecular disulfide bonds. It is very difficult to obtain a mature protein similar to the natural form by separately expressing the two genes described above using recombinant expression technology and subsequently assembling them, because the two genes are expressed separately, and the assembly is performed as an independent process, thereby making it difficult to control. The present invention provides a fusion protein obtained by expressing the two chains in a fused form, which can significantly reduce the difficulty of expression. The resulting recombinant Fel d 1 protein includes the complete primary structures of both Chain 1 and Chain 2 of native Fel d 1 and exhibits favorable biological activity. Compared with natural cat allergen extracts, the recombinant Fel d 1 protein avoids batch-to-batch variability in content and activity of major allergens from different sources, provides more stable and controllable processes and quality, and prevents degradation of the major allergen or induction of additional sensitization caused by interactions among other components in natural extracts. It meets the requirements for safety, efficacy, and quality control of modern biopharmaceuticals, and can be used for the treatment and diagnosis of allergic diseases caused by cat allergens, such as allergic rhinitis and asthma. It improves the precision of desensitization immunotherapy and the accuracy of cat allergy diagnosis, and has promising pharmaceutical prospects.

Preferably, the nucleotide sequence encoding the Fel d 1 protein is as set forth in SEQ ID NO: 3 or SEQ ID NO: 4, corresponding to the recombinant proteins Fel d 1-1 and Fel d 1-2, respectively. The sequences of Chain 1 and Chain 2 are codon-optimized for an Escherichia coli expression system, thereby facilitating expression of the two chains of Fel d 1 in the Escherichia coli expression system.

A second objective of the present invention is to provide a Fel d 4 mutant or a derivative thereof for use in the treatment of allergic diseases caused by cat allergens, where the Fel d 4 mutant includes a deletion of, or a substitution at, the cysteine residue at position 139 of native Fel d 4 (based on the native mature peptide). In the case of amino acid substitution, the cysteine residue at position 139 may be replaced with any one or more amino acids selected from G, A, V, L, I, Y, S, K, R, H, F, W, M, and T; preferably any one of G, A, V, L, I, Y, F, and W; more preferably any one of G, A, V, L, and I. The native Fel d 4 is a native mature peptide, the amino acid sequence of which is as set forth in SEQ ID NO: 7.

The Fel d 4 mutant also includes proteins obtained by introducing mutations into isoallergens or variants of the native Fel d 4 described herein, where the mutation site corresponds to position 139 of native Fel d 4 as set forth in SEQ ID NO: 7.

In specific embodiments of the present invention, the recombinant Fel d 4 protein is prepared by expression in an Escherichia coli system. Compared with the amino acid sequence as set forth in SEQ ID NO: 7, the recombinant protein further includes an additional N-terminal formylmethionine residue. In addition, based on the general knowledge described above, when expressed in a eukaryotic system, the recombinant protein may further include an additional N-terminal methionine relative to the amino acid sequence as set forth in SEQ ID NO: 7.

The derivatives include not only the full-length protein of the Fel d 4 mutant described herein, but also fragments thereof, as well as proteins obtained by further mutation based on the Fel d 4 mutant, fusion proteins, and various modified forms thereof.

Native Fel d 4 (nFel d 4) is a type IV cat allergen and a member of lipocalin family. One study has shown that the positive rate of serum IgE specific to Fel d 4 was 62.96% among patients with cat hair allergy (17/27), which is relatively high, indicating its importance in the diagnosis and treatment of allergic diseases caused by cat allergens (Smith W, et al., Clin Exp Allergy, 2004).

In the Fel d 4 mutant of the present invention, the amino acid at position 139 of native Fel d 4 (or the corresponding position in its isoallergens or variants) is deleted or substituted with another amino acid. Compared with the wild-type recombinant Fel d 4 protein, the mutant exhibits significantly improved molecular weight homogeneity (from heterogeneous to highly homogeneous), markedly increased HPLC-RP purity (from below 70% to above 95%), and correct disulfide bond pairing consistent with theoretical expectations, indicating a correct primary structure. The mutant is superior in protein homogeneity and purity and also demonstrates good in vitro and in vivo biological activity. Compared with natural cat allergen extracts, the Fel d 4 mutant avoids batch-to-batch variability in the content and activity of major allergens from different sources, provides more stable and controllable processes and quality, and prevents degradation of major allergens or induction of additional sensitization caused by interactions among other components in natural extracts. It meets the requirements for safety, efficacy, and quality control of modern biopharmaceuticals, and can be used for the treatment and diagnosis of allergic diseases caused by cat allergens, such as allergic rhinitis and asthma. It improves the precision of desensitization immunotherapy and the accuracy of cat allergy diagnosis, and has promising pharmaceutical prospects.

The present invention further provides a nucleotide sequence encoding the above-mentioned Fel d 4 mutant or a derivative thereof, where a preferred nucleotide sequence is as set forth in SEQ ID NO: 9.

A third objective of the present invention is to provide a Fel d 7 mutant or a derivative thereof for use in the treatment of allergic diseases caused by cat allergens, where the Fel d 7 mutant includes a deletion of, or a substitution at, the cysteine residue at position 101 of native Fel d 7 (based on the mature peptide). In the case of amino acid substitution, the cysteine residue at position 101 may be replaced with any one or more amino acids selected from G, A, V, L, I, Y, S, K, R, H, F, W, M, and T; preferably any one of G, A, V, L, I, Y, F, and W; more preferably any one of G, A, V, L, and I. The native Fel d 7 is a native mature peptide, the amino acid sequence of which is as set forth in SEQ ID NO: 10.

The Fel d 7 mutant also includes proteins obtained by introducing mutations into isoallergens or variants of native Fel d 7 described herein, where the mutation site corresponds to position 101 of native Fel d 7 as set forth in SEQ ID NO: 10.

In specific embodiments of the present invention, the recombinant Fel d 7 protein is prepared by expression in an Escherichia coli system. Compared with the amino acid sequence as set forth in SEQ ID NO: 10, the recombinant protein further includes an additional N-terminal formylmethionine residue. In addition, based on the general knowledge described above, when expressed in a eukaryotic system, the recombinant protein may further include an additional N-terminal methionine relative to the amino acid sequence as set forth in SEQ ID NO: 10.

The derivatives include not only the full-length protein of the Fel d 7 mutant described herein, but also fragments thereof, as well as proteins obtained by further mutation based on the Fel d 7 mutant, fusion proteins, and various modified forms thereof.

Native Fel d 7 (nFel d 7) is a type VII cat allergen and a member of lipocalin family. It is an important allergen in cat-allergic individuals in certain regions and primarily exhibits cross-reactivity with the dog allergen Can f 1 and the horse allergen Equ c 1, thereby having significant importance in the diagnosis and treatment of allergic diseases caused by cat allergens or cross-allergic diseases involving cats and dogs (D. Apostolovic et al., Allergy, 2016).

In the Fel d 7 mutant of the present invention, the amino acid at position 101 of native Fel d 7 (or the corresponding position in its isoallergens or variants) is deleted or substituted with another amino acid. Compared with the wild-type recombinant Fel d 7 protein, the mutant exhibits significantly improved molecular weight homogeneity, disulfide bond pairing consistent with theoretical expectations, and high similarity to the native protein in terms of amino acid sequence, disulfide bonds, and molecular weight. The mutant retains good allergenic immunological activity and is superior in protein homogeneity, purity, and quality control. Compared with natural cat allergen extracts, the Fel d 7 mutant avoids batch-to-batch variability in the content and activity of major allergens from different sources, provides more stable and controllable processes and quality, and prevents degradation of major allergens or induction of additional sensitization caused by interactions among other components in natural extracts. It meets the requirements for safety, efficacy, and quality control of modern biopharmaceuticals, and can be used for the treatment and diagnosis of allergic diseases caused by cat allergens, such as allergic rhinitis and asthma. It improves the precision of desensitization immunotherapy and the accuracy of cat allergy diagnosis, and has promising pharmaceutical prospects.

The present invention further provides a nucleotide sequence encoding the above-mentioned Fel d 7 mutant or a derivative thereof, where a preferred nucleotide sequence is as set forth in SEQ ID NO: 12.

The present invention further provides an expression vector including a codon-optimized gene encoding the above-described recombinant cat allergen protein, including expression vectors based on a T7 promoter, such as pET32a, pET26b, pET28a, and pDEST14, as well as expression vectors based on a temperature-inducible PL-PR promoter, such as pBV220.

The present invention further provides an Escherichia coli host including the above-mentioned recombinant expression vector. When used in combination with expression vectors based on the PL-PR promoter, any genetically engineered Escherichia coli strain may be used, preferably BL21 (DE3), BL21 AI (DE3), Top10, DH5α, JM109, Rosetta (DE3), Rosetta gamiB (DE3), and BL21 (DE3) pLys hosts. When used in combination with expression vectors based on the T7 promoter, preferred Escherichia coli strains include BL21 (DE3), BL21 AI (DE3), Rosetta (DE3), Rosetta gamiB (DE3), and BL21 (DE3) pLys.

The present invention further provides a method for expressing a recombinant cat allergen protein, including the following steps:
A. constructing a vector including a gene encoding a Fel d 1, Fel d 4, or Fel d 7 mutant or a derivative thereof as described above;
B. introducing the linearized vector obtained in step A into an Escherichia coli strain, and culturing under suitable conditions;
C. recovering and purifying the protein.

The present invention further provides a method for purifying a recombinant cat allergen protein, including the following steps:
resuspending and disrupting Escherichia coli cells obtained according to the above-mentioned method, and collecting a pellet or supernatant, where the pellet is a crude inclusion body preparation of the target protein;
when the collected material is an inclusion body, subjecting the inclusion body to preliminary purification, denaturation, and refolding treatment to obtain a refolding solution;
or, when the collected material is a supernatant, concentrating the supernatant by ultrafiltration to obtain a filtrate; and
subjecting the refolding solution or filtrate to two-step purification by ion-exchange chromatography and hydrophobic interaction chromatography to obtain a purified target protein solution. In specific embodiments, Q Sepharose Fast Flow and Phenyl Sepharose High Performance 6 (HS) are used for the two-step purification.

The present invention further provides the use of the above-mentioned recombinant cat allergen protein in the preparation of a diagnostic reagent for cat allergy diagnosis.

The present invention further provides the use of the above-mentioned recombinant cat allergen protein in the preparation of a medicament for treating an allergic disease caused by cat allergen.

The present invention further provides a composition for treating an allergic disease caused by cat allergens, including Fel d 1, Fel d 4, and Fel d 7, where the Fel d 1, Fel d 4, and Fel d 7 are natural extracted proteins or recombinant proteins, preferably the recombinant proteins described above.

The recombinant cat allergen proteins prepared according to the present invention meet the requirements for human recombinant DNA products in multiple aspects, including purity, residual process-related impurities, and molecular characterization. The purity is greater than 95%, and the proteins possess amino acid sequences, disulfide bonds, and molecular weights identical to those of the native proteins, thereby improving the quality controllability of cat allergen products. The proteins exhibit excellent reactivity with clinically allergy-positive sera in vitro and demonstrate favorable biological activity in vivo, indicating their potential application in the development of a therapeutic drug for cat allergy desensitization and a diagnostic reagent for cat allergy diagnosis. These properties provide a foundation for the precise diagnosis and treatment of allergic diseases caused by cat allergens, as well as for the standardization of cat allergen products. On the other hand, the recombinant Fel d 1 of the present invention is a fusion protein of Fel d 1 Chain 1 and Chain 2, which has been successfully expressed heterologously in Escherichia coli. While retaining the immunogenicity of the native protein, the expression difficulty is significantly reduced, and the yield of purified target protein reaches 318 mg/L. This facilitates the control of production processes and ensures the standardized preparation of recombinant Fel d 1 with high purity and homogeneity. Compared with non-mutated wild-type recombinant Fel d 4 and Fel d 7 proteins, the recombinant proteins of the Fel d 4/Fel d 7 mutants or their derivatives according to the present invention exhibit distinct advantages in terms of purity, homogeneity, and molecular characterization. Despite mutations at the relevant amino acid sites, the recombinant proteins still retain favorable allergenic biological activity, which is more conducive to the control of recombinant expression processes and the standardized and accurate preparation of recombinant proteins.

### BRIEF DESCRIPTION OF THE DRAWINGS

FIG. 1-1 shows the HPLC-SEC analysis results of purified Fel d 1-1, and FIG. 1-2 shows the HPLC-SEC analysis results of purified Fel d 1-2.
FIG. 2-1 shows the HPLC-RP analysis results of purified Fel d 1-1, and FIG. 2-2 shows the HPLC-RP analysis results of purified Fel d 1-2.
FIG. 3-1 shows the deconvoluted molecular weight spectrum of Fel d 1-1, and FIG. 3-2 shows the deconvoluted molecular weight spectrum of Fel d 1-2.
FIG. 4-1 shows the amino acid sequence coverage analysis results of Fel d 1-1, and FIG. 4-2 shows the amino acid sequence coverage analysis results of Fel d 1-2.
FIG. 5-1 shows the disulfide bond identification analysis results of Fel d 1-1, and FIG. 5-2 shows the disulfide bond identification analysis results of Fel d 1-2.
FIG. 6 is a graph showing Penh measurement results under stimulation with different concentrations of methacholine (Mch) in Example 6.
FIG. 7 is a graph showing serum antibody detection results by ELISA in Example 6.
FIG. 8-1 shows the leukocyte counts in the bronchoalveolar lavage fluid (BALF) of the negative control group and the recombinant Fel d 1 group in Example 6. FIG. 8-2 is a microscopic image of stained BALF cells from the negative control group in Example 6, and FIG. 8-3 is a microscopic image of stained BALF cells from the recombinant Fel d 1 group in Example 6.
FIG. 9 shows cytokine detection results in supernatants of in vitro-stimulated splenocytes in Example 6.
FIG. 10-1 shows the HPLC-RP analysis results of purified wild-type Fel d 4, and FIG. 10-2 shows the HPLC-RP analysis results of purified Fel d 4 mutant 2.
FIG. 11 shows the HPLC-SEC analysis results of purified Fel d 4 mutant 2.
FIG. 12 shows non-reducing SDS-PAGE analysis of purified Fel d 4, where Lanes 1-2 show non-reducing electrophoresis analysis of purified wild-type Fel d 4 (5 µl, 10 µl), and Lanes 3-4 show non-reducing electrophoresis analysis of purified Fel d 4 mutant 2 (10 µl, 5 µl).
FIG. 13 shows the deconvoluted molecular weight spectrum of Fel d 4 mutant 2.
FIG. 14 shows the amino acid sequence coverage analysis results of Fel d 4 mutant 2.
FIG. 15 shows the disulfide bond identification analysis results of Fel d 4 mutant 2.
FIG. 16 is a graph showing serum antibody detection results by ELISA in Example 12.
FIG. 17-1 is a microscopic image of stained BALF cells from the negative control group, and FIG. 17-2 is a microscopic image of stained BALF cells from the Fel d 4 mutant group.
FIG. 18 shows cytokine detection results in supernatants of in vitro-stimulated splenocytes in Example 12.
FIG. 19-1 shows the HPLC-RP analysis results of purified wild-type Fel d 7, and FIG. 19-2 shows the HPLC-RP analysis results of purified Fel d 7 mutant 1.
FIG. 20 shows the HPLC-SEC analysis results of purified Fel d 7 mutant 1.
FIG. 21 shows non-reducing SDS-PAGE analysis results of purified Fel d 7 mutant 1 and wild-type Fel d 7, where Lane 1 shows non-reducing electrophoresis analysis of purified wild-type Fel d 7 (10 µl), and Lane 2 shows non-reducing electrophoresis analysis of purified Fel d 7 mutant 1 (10 µl).
FIG. 22-1 shows the deconvoluted molecular weight spectrum of wild-type Fel d 7, and FIG. 22-2 shows the deconvoluted molecular weight spectrum of Fel d 7 mutant 1.
FIG. 23 shows the amino acid sequence coverage analysis results of Fel d 7 mutant 1.
FIG. 24 shows the disulfide bond identification analysis results of Fel d 7 mutant 1.
FIG. 25 is a graph showing Penh measurement results under stimulation with different concentrations of methacholine (Mch) in Example 17.
FIG. 26 is a graph showing the detection results of specific IgE levels in serum by ELISA in Example 17.
FIG. 27-1 is a microscopic image of stained BALF cells from the negative control group, and FIG. 27-2 is a microscopic image of stained BALF cells from the Fel d 7 group.
FIG. 28 shows cytokine detection results in supernatants of in vitro-stimulated splenocytes in Example 17.

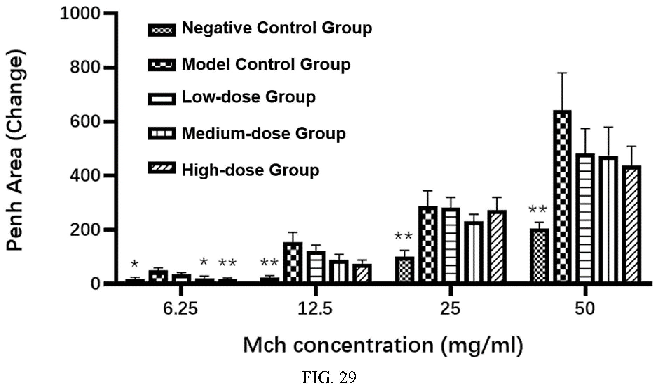

FIG. 29 is a graph showing Penh measurement results under stimulation with different concentrations of methacholine (Mch) in Example 18.

### DETAILED DESCRIPTION OF THE EMBODIMENTS

The present invention is further described below with reference to specific embodiments. It should be understood that these embodiments are provided for illustrative purposes only and are not intended to limit the scope of the present invention.

### Example 1: Design of Recombinant Fel d 1 and Construction of Expression Strains

The mature peptide sequences of Fel d 1 Chain 1 and Chain 2 were obtained according to GenBank accession numbers AAC37318 and AAC41616, respectively. Fel d 1-1 was designed in the order of N-Chain 1 mature peptide-Chain 2 mature peptide-C. The gene encoding Fel d 1-1 was codon-optimized for expression in an Escherichia coli expression system and synthesized by Nanjing GenScript Biotechnology Co., Ltd. Based on the synthesized coding DNA sequence of Fel d 1-1, the coding DNA sequence of Fel d 1-2 was obtained by PCR, where the construction strategy was N-Chain 2-Chain 1-C. An NdeI restriction site was introduced at the 5' end, and an XhoI restriction site was introduced at the 3' end, of the coding DNA sequences of both Fel d 1-1 and Fel d 1-2.

Recombinant expression of Fel d 1 according to the present invention may be carried out using expression vectors based on a T7 promoter, such as pET32a, pET26b, pET28a, and pDEST14, or using expression vectors based on a temperature-inducible PL-PR promoter, such as pBV220. When using expression vectors based on the PL-PR promoter, any genetically engineered Escherichia coli strain may be selected, including but not limited to BL21 (DE3), BL21 AI (DE3), Top10, DH5α, JM109, Rosetta (DE3), Rosetta gamiB (DE3), and BL21 (DE3) pLys. When using expression vectors based on the T7 promoter, suitable Escherichia coli strains include BL21 (DE3), BL21 AI (DE3), Rosetta (DE3), Rosetta gamiB (DE3), and BL21 (DE3) pLys. In this example, the pET26b vector and BL21 (DE3) host strain were used for illustration.

The DNA sequences of Fel d 1-1 and Fel d 1-2 were respectively cloned into the pET26b vector to construct expression plasmids pET26b-Fel d 1-1 and pET26b-Fel d 1-2. The two recombinant expression plasmids were then separately transformed into the BL21 (DE3) host strain to obtain the expression strains BL21 (DE3)-pET26b-Fel d 1-1 and BL21 (DE3)-pET26b-Fel d 1-2.

**Table 1**

| **Protein (or Peptide Chain) Name** | **Primary Structure** | **Amino Acid Sequence ID** | **DNA Sequence ID** |
|---|---|---|---|
| Fel d 1-1 | N-Chain 1-Chain 2-C | SEQ ID NO: 1 | SEQ ID NO: 3 |
| Fel d 1-2 | N-Chain 2-Chain 1-C | SEQ ID NO: 2 | SEQ ID NO: 4 |

### Example 2: Recombinant Expression of Fel d 1

Expression of Fel d 1-1 in the pET26b vector and BL21 (DE3) host strain is described below. Fel d 1-2 was expressed using the same method.
1. BL21(DE3)-pET26b-Fel d 1-1 strain was streaked from a glycerol stock onto an LB agar plate containing 50 µg/mL and incubated at 37°C for 12 h;
2. A single colony was picked from the agar plate and inoculated into 10 mL of TB liquid medium, and cultured at 37°C with shaking at 220 rpm for 8 h. The resulting culture was used as the primary seed culture;
3. The primary seed culture was inoculated at 5% (v/v) into ten flasks each containing 500 mL of TB liquid medium, followed by incubation at 37°C with shaking at 220 rpm; when the OD reached 0.8, IPTG was added at 0.5 mM to induce expression; after induction for 6 h, the cells were harvested by centrifugation for subsequent use.

### Example 3: Extraction and Purification of Recombinant Fel d 1

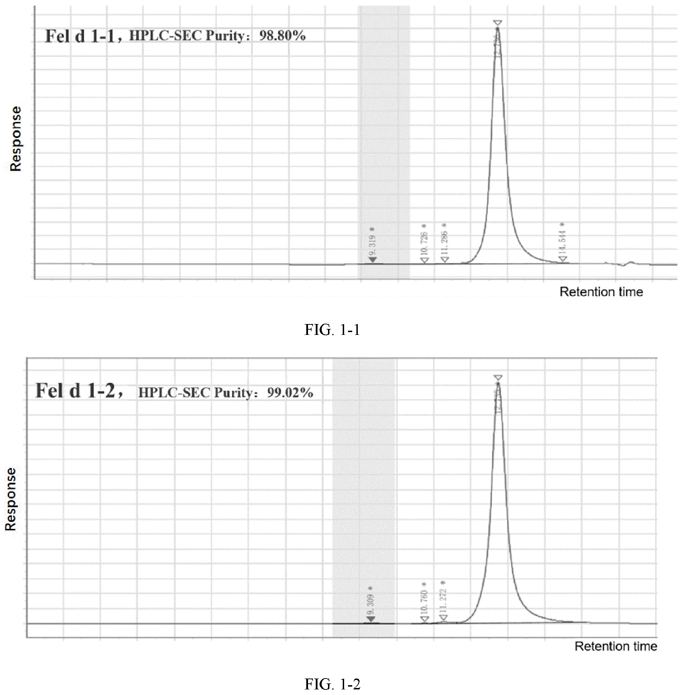

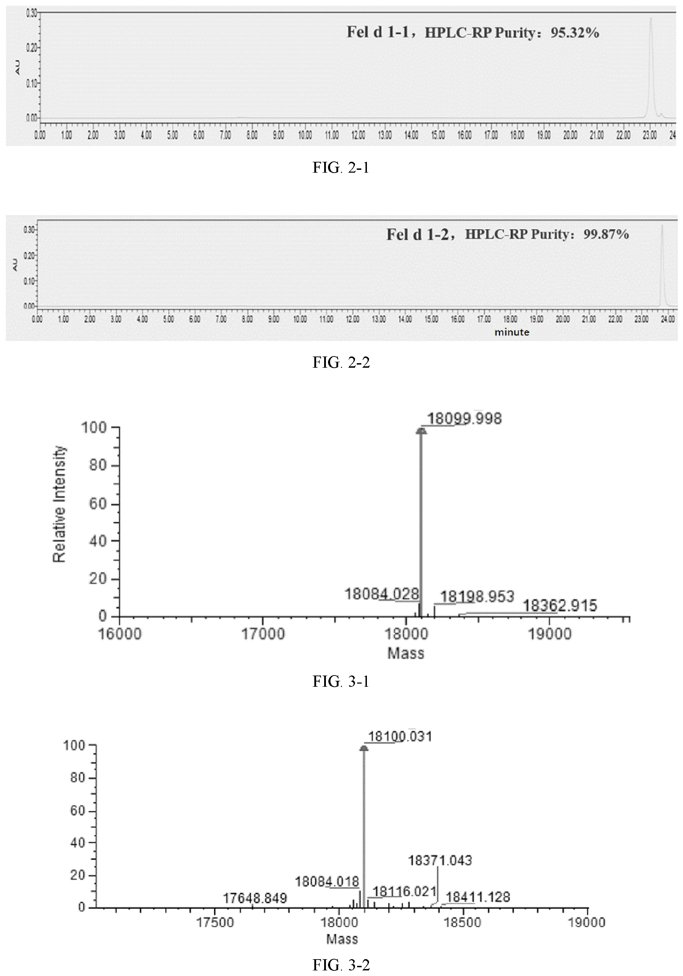

Extraction and purification of Fel d 1-1 are described below. The purified sample of Fel d 1-2 was obtained using the same process.
1. Fermented cells were resuspended in a buffer of 10 mM PB + 150 mM NaCl (pH 7.4) to a concentration of 100 g/L and uniformly dispersed; cell disruption was performed using a high-pressure homogenizer at 900 ± 50 bar for three cycles, with the sample temperature maintained below 25°C throughout the process; after disruption, the homogenate was centrifuged, and the resulting pellet was collected as the crude inclusion body preparation of the target protein;
2. The obtained inclusion bodies were resuspended in 10 mM PB + 1.5 M urea + 0.5% Triton X-100 + 150 mM NaCl (pH 7.4) at a ratio of 1/100 (w/v), uniformly dispersed, and stirred at 2-8°C for 8 h; the inclusion body pellet was collected by centrifugation; the above procedure was repeated twice to obtain partially purified inclusion bodies;
3. The partially purified inclusion bodies were resuspended in a denaturation buffer at a ratio of 1 g/100 mL and stirred at room temperature for at least 1 h until the solution was substantially clear; the denaturation buffer consisted of 10 mM PB + 6 M guanidine hydrochloride + 10 mM DTT (pH 8.0); after denaturation, the supernatant was collected by centrifugation;
4. Refolding: the denatured solution was slowly added into the refolding buffer at a ratio of 1:10 with rapid stirring to ensure dispersion and to prevent local overconcentration of protein; the addition of the denatured solution was carried out over 10 h, and the temperature of the refolding solution was maintained at 15°C during addition; after completion of the addition, refolding was allowed to proceed for 36 h. The refolding buffer consisted of 20 mM Tris + 0.5 M urea + 3 mM reduced glutathione + 1 mM oxidized glutathione (pH 8.0);
5. After refolding, the solution was centrifuged and filtered to remove insoluble materials, followed by ultrafiltration using a 5 kDa molecular weight cut-off membrane to concentrate the sample and exchange the buffer to 10 mM PB (pH 8.0); the resulting solution was retained for subsequent use;
6. First-step purification: the refolding solution after buffer exchange was purified using Q Sepharose Fast Flow; a linear gradient of NaCl was applied to reach 0.5 M over 10 column volumes, and the target protein peak was collected; the binding buffer was 10 mM PB (pH 8.0), and the elution buffer was 10 mM PB + 1 M NaCl (pH 8.0);
7. Second-step purification: ammonium sulfate was added to the sample from the first-step purification to a final concentration of 0.5 M, and the pH was adjusted to 7.5; purification was performed using a Phenyl Sepharose High Performance 6 (HS) column; a linear gradient was applied over 10 column volumes, reaching 100% elution buffer, and the target protein peak was collected; the binding buffer was 10 mM PB + 0.5 M ammonium sulfate (pH 7.4), and the elution buffer was 10 mM PB (pH 7.4);
8. The target protein peaks collected from the two chromatography steps were subjected to ultrafiltration and buffer exchange into 20 mM PB (pH 7.0); after sterile filtration, the target protein bulk solution was obtained; based on determination of the protein content in the bulk solution, it was calculated that at a 3 L fermenter scale, a single batch of inclusion bodies after refolding and purification yielded 954 mg of the target protein, corresponding to a yield of 318 mg/L;
9. The final samples were analyzed by HPLC-SEC and HPLC-RP, and the results are shown in FIG. 1 and FIG. 2, respectively. As shown in FIG. 1 and FIG. 2, both Fel d 1-1 and Fel d 1-2 achieved a purity of greater than 95% using two analytical methods based on different separation principles, meeting general purity requirements for recombinant protein pharmaceuticals.

### Example 4: Primary Structure Analysis and Molecular Weight Determination of Recombinant Fel d 1

LC-MS molecular weight analysis can accurately reflect whether the primary sequence of a biological macromolecule is correct, including whether the N- and C-terminal sequences are intact, and whether post-translational modifications such as glycosylation, oxidation, and deamidation are present. It is one of the most important analytical methods for biological macromolecules.

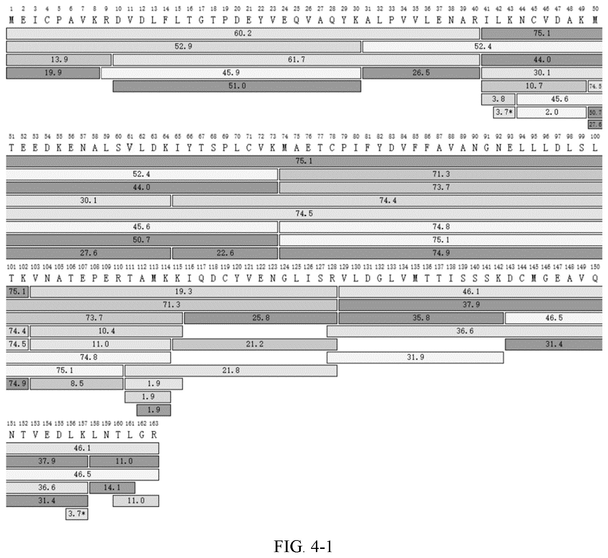

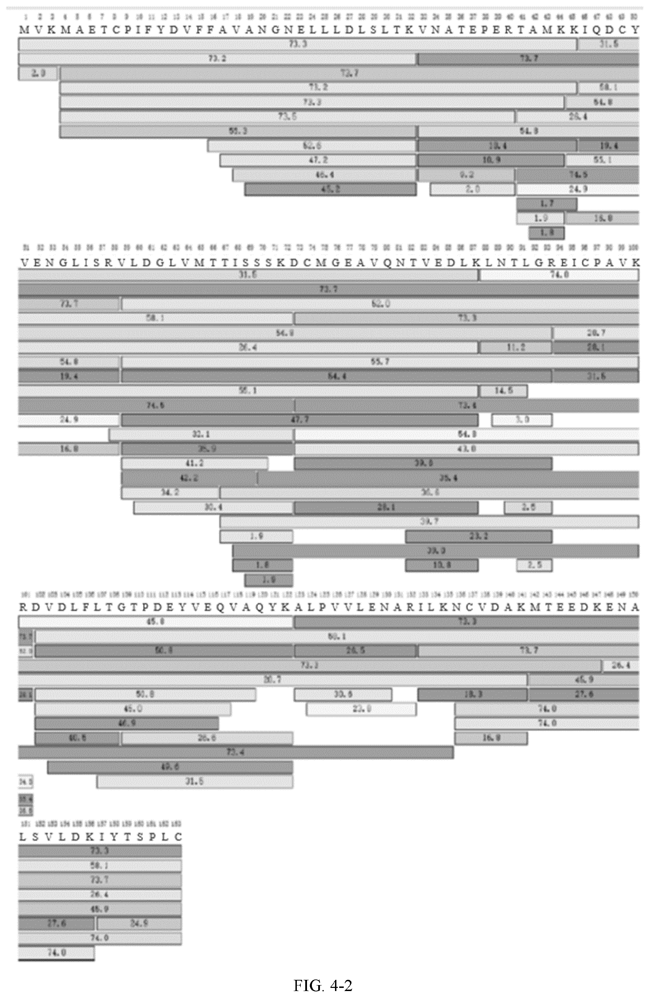

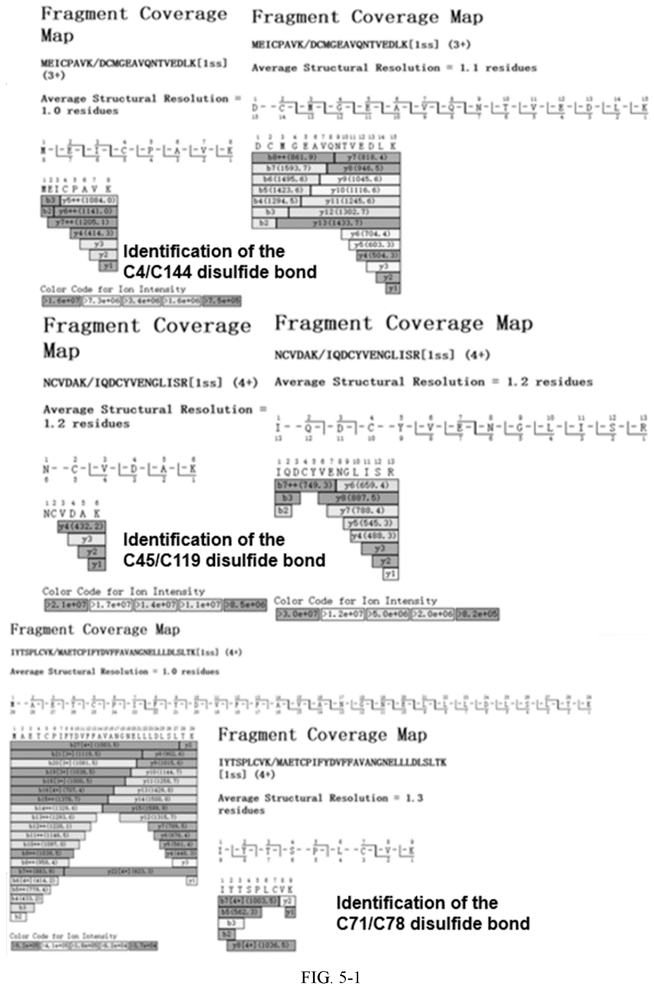

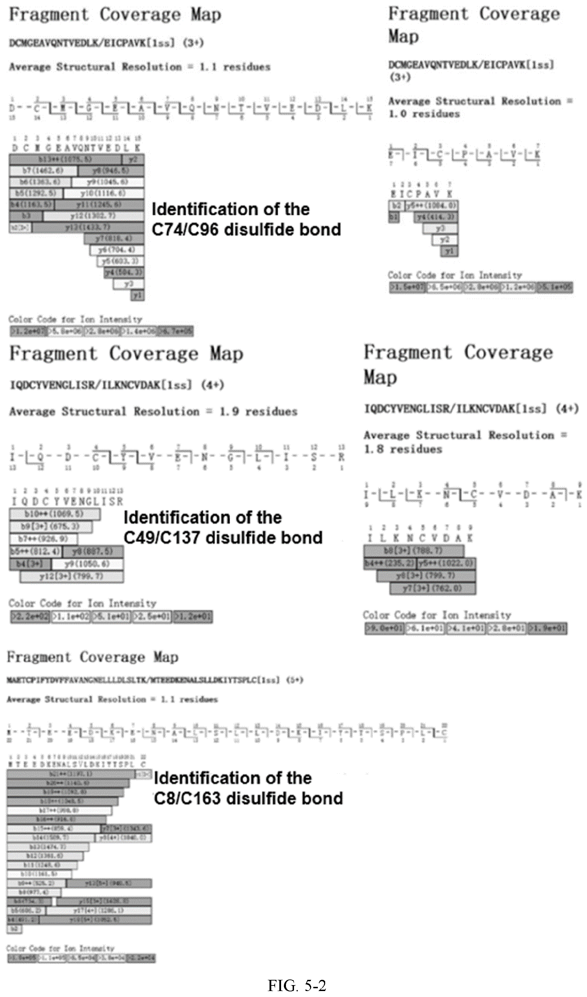

The purified recombinant Fel d 1-1 and Fel d 1-2 proteins were subjected to LC-MS molecular weight determination and primary structure analysis using a Thermo Scientific^{™} high-resolution mass spectrometry system. The results are shown in FIG. 3-FIG. 5.

As shown in FIG. 3-FIG. 5, the molecular weights of Fel d 1-1 and Fel d 1-2 are consistent with the expected values (theoretical molecular weight: 18,100 Da), and the amino acid sequence coverage of both reached 100%, indicating that the primary structures of both proteins are correct. In addition, disulfide bond pairing analysis of Fel d 1-1 and Fel d 1-2 showed that the disulfide bond patterns identified in both design formats were consistent with theoretical expectations, thereby supporting the formation of higher-order structures.

### Example 5: Serum Reactivity Analysis of Recombinant Fel d 1

In this example, serum reactivity of recombinant Fel d 1 prepared in the present invention was evaluated using clinical cat allergy sera collected from Jiangsu Provincial People's Hospital between March and May 2023. The assay procedure was as follows:
1. Coating: Recombinant Fel d 1 protein was diluted to 1 µg/mL using 0.05 M carbonate coating buffer (pH 9.6); the diluted protein was added to a microplate at 100 µL per well and incubated at 2-8°C overnight;
2. Blocking: On the following day, the microplate was washed once with PBST; blocking was performed by adding 2% BSA in PBST at 200 µL per well, followed by incubation at 37°C for 2 h;
3. Sample Preparation: Clinical cat allergy positive and negative sera were diluted with 2% BSA in PBST (dilution factors are shown in Tables 2 and 3); 2% BSA in PBST was used as the blank background control; after removal of liquid from the wells, 100 µL of each diluted sample was added per well and incubated at 37°C with shaking at 300 rpm for 2 h;
4. Detection: The plate was washed three times with PBST; mouse anti-human IgE-HRP secondary antibody diluted at 1:1500 was added at 100 µL per well, followed by incubation at 37°C with shaking at 300 rpm for 1 h;
5. Color Development, Termination, and Reading: The plate was washed three times with PBST; TMB substrate solution (Type VII) was added at 100 µL per well; after incubation at 37°C for 15 min, 50 µL of 2 M sulfuric acid was added to each well to terminate the reaction; the microplate was immediately read at 450 nm using a microplate reader (Thermo Multiskan GO);
6. Result Interpretation and Analysis: The microplate reader readings reflect the binding level between Fel d 1 and allergen-specific IgE in serum. The results in Table 2 show that both Fel d 1-1 and Fel d 1-2 exhibited reactivity with different clinical cat allergy positive sera to varying degrees. At the same dilution factors, both proteins showed comparable reactivity across different sera. The reactivity exhibited a positive correlation with the clinical diagnostic values of the corresponding positive sera, consistent with the conclusion that Fel d 1 is a major cat allergen.

In Table 3, 1.5-fold of the blank background value was used as the cutoff threshold. The highest serum dilution exceeding this cutoff was defined as the reported reactivity result. A higher dilution factor indicates a higher level of specific IgE reacting with Fel d 1 in the clinical cat allergy serum. The results showed that recombinant Fel d 1-1 exhibited varying degrees of reactivity with 11 clinical cat allergy positive sera, while showing no reactivity with negative sera, supporting the conclusion that Fel d 1 is a major cat allergen. In addition, three clinical serum samples (RY179, RY187, and RY195) showed no reactivity with recombinant Fel d 1-1, despite being clinically reported as positive. This suggests that these sera may not contain Fel d 1-specific IgE but instead contain IgE specific to cat allergens other than Fel d 1. Accordingly, the corresponding patients may exhibit allergic responses to cat allergens other than Fel d 1. The reactivity results of recombinant Fel d 1-2 were consistent with the above findings.

In summary, the recombinant Fel d 1 proteins of the present invention exhibit reactivity with clinical cat allergy positive sera and are capable of binding Fel d 1-specific IgE in serum. The reactivity correlates with clinical diagnostic results. Recombinant Fel d 1 of the present invention in different formats exhibits similar reactivity profiles to clinical sera.

**Table 2**

| **Clinical Serum Samples** | **Clinical Diagnostic Value (KUA/L) (quantified using Phadia 250)** | **Dilution Factor** | **Reading at 450 nm** | |
|---|---|---|---|---|
| | | | **Fel d 1-1** | **Fel d 1-2** |
| 8803 | 1.88 | 10 | 0.7718 | 0.8214 |
| 8779 | 1.19 | 10 | 0.3392 | 0.3404 |
| 4606 | 1.91 | 10 | 0.0815 | 0.0750 |
| 1363 | 4.55 | 10 | 1.0184 | 0.8842 |
| SZ114 | 5.62 | 10 | 3.2776 | 2.8754 |
| SZ15 | 5.62 | 10 | 2.8522 | 2.8285 |
| 8773 | 11.7 | 50 | 1.6003 | 1.3889 |
| CZ224 | 13.2 | 50 | 1.6920 | 1.4791 |
| SZ11 | 16.2 | 50 | 2.0571 | 1.5868 |
| SZ28 | 20.1 | 50 | 1.8857 | 1.6051 |
| SZ19 | 60.5 | 50 | 3.8583 | 3.9228 |
| Negative Serum | 0 | 10 | 0.0908 | 0.0662 |

**Table 3**

| **Clinical Serum Samples** | **Clinical Diagnostic Value (kUA/L) (quantified using Phadia 250)** | **Fel d 1-1 Reactivity Result (Dilution Factor)** |
|---|---|---|
| RY33 | 1.33 | 100 |
| RY62 | 2.99 | 100 |
| RY100 | 48.9 | 500 |
| RY101 | 0.79 | 20 |
| RY126 | 4.02 | 100 |
| RY161 | 2.22 | 100 |
| RY166 | 0.79 | 20 |
| RY179 | 9.17 | 0 |
| RY183 | 3.39 | 20 |
| RY185 | 0.82 | 20 |
| RY187 | 0.65 | 0 |
| RY195 | 9.42 | 0 |
| RY201 | 7.3 | 100 |
| RY215 | 19.6 | 20 |
| Negative Serum | 0 | 0 |

### Example 6: Evaluation of In Vivo Sensitization and Desensitization Efficacy of Recombinant Fel d 1

### 1. Materials

Female BALB/c mice, aged 6-7 weeks at the time of receipt, were obtained from Changzhou Cavens Laboratory Animal Co., Ltd.

Recombinant Fel d 1 solution prepared in the present invention (Fel d 1-1 as an example).

Aluminum hydroxide adjuvant, provided by Thermo Fisher Scientific.

### 2. Preparation of Allergen Samples

Sensitization sample: A Fel d 1-1 solution at a concentration of 0.0067 mg/mL was mixed with aluminum hydroxide adjuvant at a volume ratio of 3:1 to prepare the sensitization sample. The mixture was thoroughly shaken prior to injection. Challenge sample: A Fel d 1-1 solution at a concentration of 0.05 mg/mL was used as the challenge sample.

### 3. Administration Regimen

### 3.1 Sensitization Phase

Sixteen mice were randomly divided into two groups (n = 8 per group) according to body weight. Grouping and dosing details are shown in Table 4. The recombinant Fel d 1 group was subcutaneously injected with the sensitization sample at 1 µg per mouse per dose. The negative control group received an equal volume of blank sample. Administration was performed once weekly for a total of three doses.

**Table 4 Grouping and Administration**

| Group Name | Model-Inducing Agent | Sensitization Dose (µg/mouse/time) | Challenge Dose (µg/mouse/time) |
|---|---|---|---|
| Negative Control Group | PBS | 0 | 0 |
| Recombinant Fel d 1 Group | Fel d 1-1 | 1 | 2 |

### 3.2 Challenge Phase

One week after the final sensitization, mice in the recombinant Fel d 1 group were intranasally challenged with the challenge sample at 40 µL per mouse per dose, once daily for 7 consecutive days. The negative control group received an equal volume of blank sample. Details are shown in Table 4.

### 4. Evaluation Parameters

### 4.1 Penh:

24 hours after the final challenge, Penh values were measured in all mice using a non-invasive whole-body plethysmography (WBP) system at methacholine (Mch) concentrations of 0, 6.25, 12.5, 25, and 50 mg/mL. Penh reflects airway resistance; higher Penh values indicate increased airway responsiveness and more severe airway symptoms.

### 4.2 Serum Antibodies

48 hours after the final challenge, whole blood was collected via orbital bleeding. After standing at room temperature for 2-3 h, the collected whole blood samples were centrifuged at 3500 rpm for 10 min to obtain serum. Serum antibodies were measured by ELISA, using Fel d 1-1 as the coating antigen.

### 4.3 White Blood Cell Counts in BALF

Bronchoalveolar lavage fluid (BALF) was collected, and three lavage fractions were pooled and centrifuged. The supernatant was discarded, and the cell pellet was resuspended in 1×PBS buffer. After thorough mixing, the cells were stained in accordance with the instructions of the Wright-Giemsa stain kit. Under an upright microscope at 200× magnification, three random fields were selected, and stained cells were counted using ImageJ software to obtain average values.

### 4.5 Cytokines in supernatants of in vitro-stimulated splenocytes

Splenocytes were isolated from mice in each group under sterile conditions and stimulated with the challenge sample. After co-culture with the challenge sample for 144 hours, the supernatants were collected. The levels of IL-4, IL-5, IL-10, and IL-13 in the supernatants of in vitro-stimulated splenocytes were measured using a cytometric bead array (CBA) assay.

### 5. Results

### 5.1 Penh:

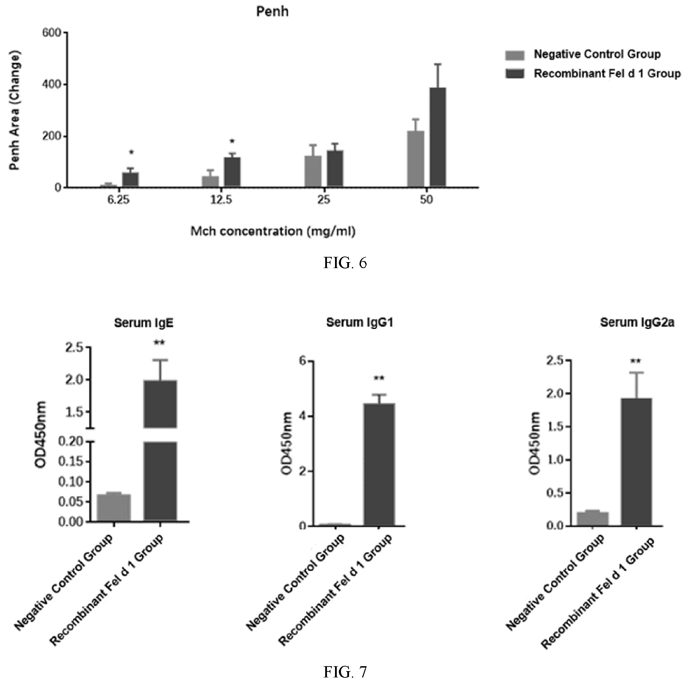

As shown in Table 5 and FIG. 6, Penh values in the recombinant Fel d 1 group were higher than those in the negative control group at all tested Mch concentrations. At 6.25 mg/mL and 12.5 mg/mL Mch, Penh values were significantly higher than those of the negative control group (p < 0.05).

**Table 5 Mouse Penh Values (Mean ± SEM)**

| Group Name | Number of Samples (n) | Penh | | | |
|---|---|---|---|---|---|
| | | Mch 6.25mg/mL | Mch 12.5mg/mL | Mch 25mg/mL | Mch 50mg/mL |
| Negative Control Group | 8 | 7.78±10.05 | 40.57±27.84 | 120.64±45.19 | 216.37±49.33 |
| Recombinant Fel d 1 Group | 8 | 56.65±19.74* | 115.25±18.3* | 142.38±28.64 | 384.34±94.54 |

| | | | | | |
|---|---|---|---|---|---|
| Note: * indicates p < 0.05 compared with the negative control group | | | | | |

### 5.2 Serum Antibodies

As shown in Table 6 and FIG. 7, the serum antibody assay results of each group indicate that,
IgE levels in the recombinant Fel d 1 group were significantly higher than those in the negative control group (p < 0.01).

IgG1 levels in the recombinant Fel d 1 group were significantly higher than those in the negative control group (p < 0.01).

IgG2a levels in the recombinant Fel d 1 group were significantly higher than those in the negative control group (p < 0.01).

**Table 6 Serum Antibody Levels (Mean ± SEM)**

| Group Name | Number of Samples (n) | IgE OD value | IgG1 OD value | IgG2a OD value |
|---|---|---|---|---|
| Negative Control Group | 8 | 0.0662±0.0056 | 0.0651±0.0107 | 0.1962±0.0314 |
| Recombinant Fel d 1 Group | 8 | 1.983±0.3262^{**} | 4.433±0.3432^{**} | 1.919±0.3983^{**} |

| | | | | |
|---|---|---|---|---|
| Note: ** indicates p < 0.01 compared with the negative control group. | | | | |

### 5.3 WBC Counts in BALF

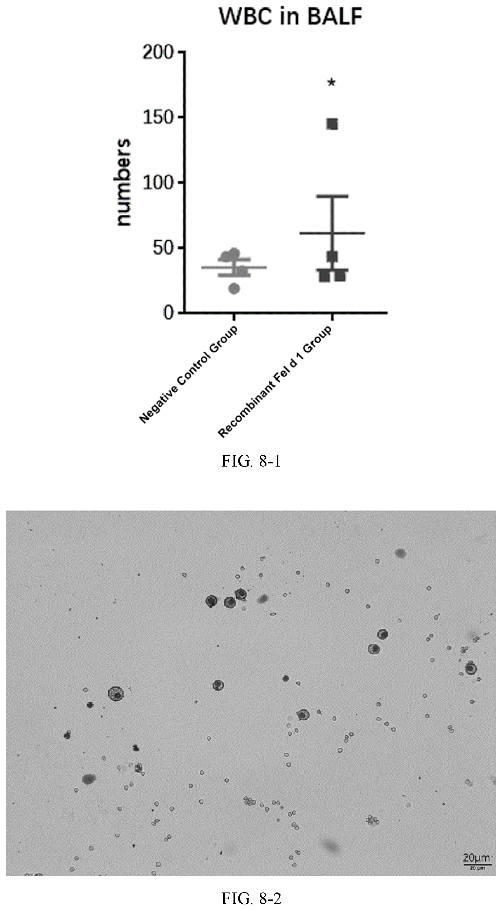

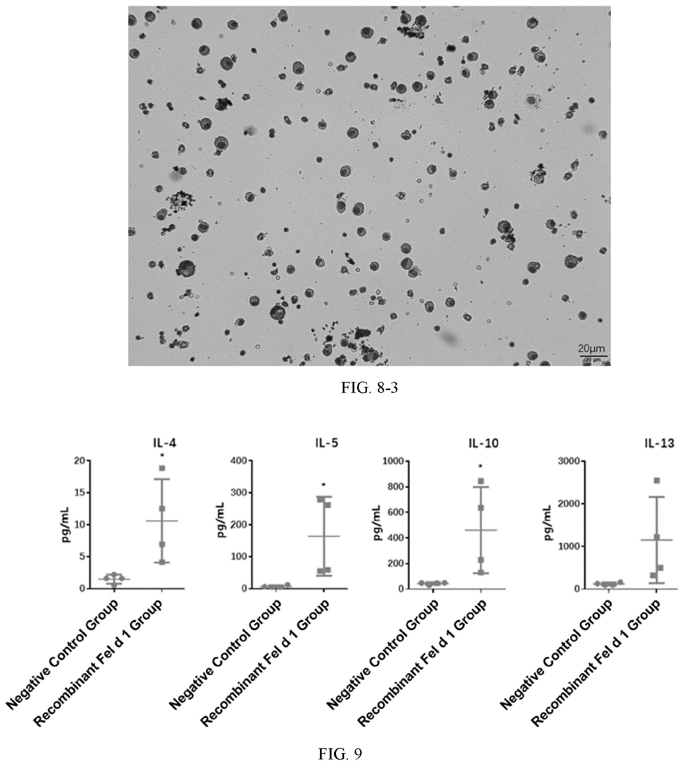

As shown in Table 7, FIG. 8-1, and the microscopic images of stained cells in BALF in FIG. 8-2 and FIG. 8-3, the WBC count in BALF of mice in the recombinant Fel d 1 group was significantly higher than that in the negative control group (p < 0.05).

**Table 7 WBC Counts in BALF (Mean ± SEM)**

| Group Name | Number of Samples (n) | WBC (cells) |
|---|---|---|
| Negative Control Group | 4 | 35.42±6.22 |
| Recombinant Fel d 1 Group | 4 | 61.58±28.14* |

| | | |
|---|---|---|
| Note: * indicates p < 0.05 compared with the negative control group | | |

### 5.4 Cytokines in supernatants of in vitro-stimulated splenocytes

As shown in Table 8 and FIG. 9, Th2-type cytokines (IL-4, IL-5, IL-10, and IL-13) were increased in the recombinant Fel d 1 group. IL-4, IL-5, and IL-10 were significantly higher than those in the negative control group (p < 0.05).

**Table 8 TH2 -Type Cytokines in Supernatants of In Vitro-Stimulated Splenocytes(Mean ± SEM)**

| Group Name | Number of Samples (n) | level(pg/mL) | | | |
|---|---|---|---|---|---|
| | | IL-4 | IL-5 | IL-10 | IL-13 |
| Negative Control Group | 4 | 1.49±0.35 | 6.97±1.71 | 43.8±4.47 | 120.74±17.77 |
| Recombinant Fel d 1 Group | 4 | 10.62±3.25* | 164.02±61.75* | 460.72±168.62* | 1150.26±505.76 |

| | | | | | |
|---|---|---|---|---|---|
| Note: * indicates p < 0.05 compared with the negative control group | | | | | |

### 6. Conclusion

The results of the present study demonstrate that, in an allergic airway inflammation model established by sensitization and challenge with recombinant Fel d 1 protein, mice in the recombinant Fel d 1 group exhibited elevated levels across multiple indicators, including airway symptoms, antibody responses, target organ inflammation, and cytokines, indicating that the recombinant Fel d 1 protein of the present invention possesses immunogenicity in vivo, is capable of inducing allergic responses, increasing allergen-specific antibody levels, and stimulating immune responses. Accordingly, the recombinant Fel d 1 protein of the present invention has potential for use as an active ingredient in cat allergen immunotherapy or as a component in diagnostic reagents for cat allergy diagnosis, thereby enabling application in the diagnosis and treatment of cat allergic diseases.

### Example 7: Design of Fel d 4 Mutants and Construction of Expression Strains

Native Fel d 4 may exist as multiple isoallergens or variants. In this example, the nFel d 4 sequence with GenBank accession No. AAS77253 is used as a representative.

The mature peptide sequence of nFel d 4 was obtained according to the GenBank accession and is shown in SEQ ID NO: 7. The corresponding DNA sequence was obtained by reverse translation and was codon-optimized for an Escherichia coli expression system by Nanjing GenScript Biotechnology Co., Ltd., followed by synthesis to obtain the coding DNA sequence of nFel d 4, as shown in SEQ ID NO: 8. A deletion or amino acid substitution mutation at position 139 of nFel d 4 was introduced by PCR (mutation information is shown in Table 9). NdeI and XhoI restriction sites were introduced at the 5' end and 3' end of the mutant coding sequences, respectively. Taking Mutant 2 as an example, its DNA sequence is shown in SEQ ID NO: 9.

Recombinant expression of Fel d 4 according to the present invention may be carried out using expression vectors based on a T7 promoter, such as pET32a, pET26b, pET28a, and pDEST14, or expression vectors based on a temperature-inducible PL-PR promoter, such as pBV220. When using expression vectors based on the PL-PR promoter, any genetically engineered Escherichia coli strain may be selected, including but not limited to BL21 (DE3), BL21 AI (DE3), Top10, DH5α, JM109, Rosetta (DE3), Rosetta gamiB (DE3), and BL21 (DE3) pLys. When using expression vectors based on the T7 promoter, suitable Escherichia coli strains include BL21 (DE3), BL21 AI (DE3), Rosetta (DE3), Rosetta gamiB (DE3), and BL21 (DE3) pLys. In this example, the pET26b vector and BL21 (DE3) host strain were used for illustration.

The DNA sequences of the above Fel d 4 mutants were cloned into the pET26b vector to construct pET26b-Fel d 4 mutant expression plasmids, which were then transformed into the BL21 (DE3) host strain to obtain BL21 (DE3)-pET26b-Fel d 4 mutant expression strains.

**Table 9 Summary of Mutation Information for Each Sample**

| **Protein Name** | **Mutation at position 139** |
|---|---|
| nFel d 4 | No mutation |
| Fel d 4 mutant 1 | Deletion of the original C at position 139 |
| Fel d 4 mutant 2 | C139G |
| Fel d 4 mutant 3 | C139A |
| Fel d 4 mutant 4 | C139V |
| Fel d 4 mutant 5 | C139L |
| Fel d 4 mutant 6 | C139I |
| Fel d 4 mutant 7 | C139Y |
| Fel d 4 mutant 8 | C139S |
| Fel d 4 mutant 9 | C139R |
| Fel d 4 mutant 10 | C139H |
| Fel d 4 mutant 11 | C139F |
| Fel d 4 mutant 12 | C139W |
| Fel d 4 mutant 13 | C139M |
| Fel d 4 mutant 14 | C139T |
| Fel d 4 mutant 15 | C139K |

### Example 8: Recombinant Expression of Fel d 4 and Its Mutants

Expression of Fel d 4 mutant 2 in the pET26b vector and BL21 (DE3) host strain is described below. The same method was used for the expression of nFel d 4 and other mutants.
1. BL21 (DE3)-pET26b-Fel d 4 mutant 2 strain was streaked from a glycerol stock onto an LB agar plate containing 50 µg/mL and incubated at 37°C for 12 h;
2. A single colony was picked from the agar plate and inoculated into 10 mL of TB liquid medium, and cultured at 37°C with shaking at 220 rpm for 8 h; the resulting culture was used as the primary seed culture;
3. The primary seed culture was inoculated at a ratio of 1-2% into ten flasks each containing 500 mL of TB liquid medium, followed by incubation at 37°C with shaking at 220 rpm; when the OD reached 0.8, expression was induced by adding 0.5 mM IPTG; after induction for 4 h, the cells were harvested by centrifugation for subsequent use.

### Example 9: Extraction and Purification of Fel d 4 and Its Mutants

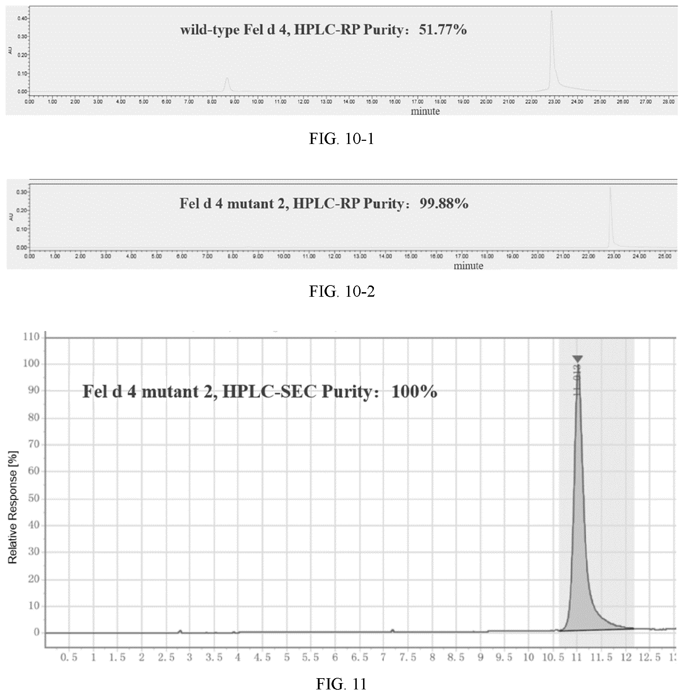

Extraction and purification of Fel d 4 mutant 2 are described below. The same process was used to obtain nFel d 4 and other mutants.
1. Fermented cells were resuspended in a buffer of 10 mM PB + 150 mM mM NaCl (pH 7.4) to a concentration of 100 g/L and uniformly dispersed; cell disruption was performed using a high-pressure homogenizer at 900 ± 50 bar for three cycles, with the sample temperature maintained below 25°C throughout the process; after disruption, the homogenate was centrifuged, and the resulting pellet was collected as the crude inclusion body preparation of the target protein;
2. The obtained inclusion bodies were resuspended in 10 mM PB + 1.5 M urea + 0.5% Triton X-100 + 150 mM NaCl (pH 7.4) at a ratio of 1/100 (w/v), uniformly dispersed, and stirred at 2-8°C for 10 h; the inclusion bodies were collected by centrifugation; the above procedure was repeated twice to obtain partially purified inclusion bodies.
3. The partially purified inclusion bodies were resuspended in denaturation buffer at a ratio of 1 g/100 mL and stirred at room temperature for at least 1 h until the solution was substantially clear; the denaturation buffer consisted of 10 mM PB + 6 M guanidine hydrochloride + 10 mM DTT (pH 8.0); after denaturation, the supernatant was collected by centrifugation;
4. Refolding: the denatured solution was slowly added to the refolding buffer at a ratio of 1:10 with rapid stirring to ensure dispersion and to prevent local overconcentration of protein; the addition of the denatured solution was carried out over 10 h, and the temperature of the refolding buffer was maintained at 15°C during addition; after completion of the addition, refolding was allowed to proceed for 36 h; the refolding buffer consisted of 20 mM Tris + 0.5 M urea + 3 mM reduced glutathione + 1 mM oxidized glutathione (pH 8.0);
5. After refolding, insoluble materials were removed by centrifugation or filtration, followed by ultrafiltration using a 10 kDa molecular weight cut-off membrane to concentrate the sample and exchange the buffer to 10 mM PB (pH 8.0); the resulting solution was retained for subsequent use;
6. First-step purification: the refolding solution after buffer exchange was purified using Q Sepharose Fast Flow; a linear gradient of NaCl was applied to reach 0.5 M over 10 column volumes, and the target protein peak was collected; the binding buffer was 10 mM PB (pH 8.0), and the elution buffer was 10 mM PB + 1 M NaCl (pH 8.0);
7. Second-step purification: ammonium sulfate was added to the sample from the first-step purification to a final concentration of 0.5 M, and the pH was adjusted to 7.4-8.0; purification was performed using a Phenyl Sepharose High Performance 6 (HS) column; a linear gradient was applied over 10 column volumes, reaching 100% elution buffer, and the target protein peak was collected; the binding buffer was 10 mM PB + 0.5 M ammonium sulfate (pH 7.4), and the elution buffer was 10 mM PB (pH 7.4);
8. The target protein peaks collected from the two chromatographic steps were subjected to ultrafiltration and buffer exchange into 20 mM PB (pH 7.0); after sterile filtration, the target protein bulk solution was obtained;
9. The final samples were analyzed by HPLC-SEC, HPLC-RP, and non-reducing SDS-PAGE, and the results are shown in FIG. 10-FIG. 12.

The results showed that both wild-type Fel d 4 and mutant 2 could achieve equivalent electrophoretic purity under the same process conditions, and no visible impurities were observed in non-reducing SDS-PAGE. However, the HPLC-RP profile of wild-type Fel d 4 exhibited a distinct impurity peak that was difficult to separate, whereas this phenomenon was not observed in the mutants. It was inferred that the purified wild-type Fel d 4 contained a related protein species with a molecular weight similar to that of the target protein but with significantly different hydrophobic properties. These results indicate that mutant 2 has advantages over wild-type Fel d 4 in terms of purity and homogeneity of the recombinant protein.

Further analysis of HPLC-RP purity for each mutant (as shown in Table 10) revealed that all mutants achieved a purity of over 95% under the same process conditions. Since all mutants contain mutations at position 139 of nFel d 4, mutation at this position is considered a key factor in improving the RP purity of Fel d 4. Compared with wild-type Fel d 4, the Fel d 4 mutants of the present invention exhibit superior purity and homogeneity of the recombinant protein, and are more advantageous for recombinant production.

**Table 10 Summary of HPLC-RP Purity of Each Sample**

| **Protein Name** | **HPLC-RP Purity** |
|---|---|
| nFel d 4 | 51.77% |
| Fel d 4 mutant 1 | 98.52% |
| Fel d 4 mutant 2 | 99.88% |
| Fel d 4 mutant 3 | 95.63% |
| Fel d 4 mutant 4 | 98.69% |
| Fel d 4 mutant 5 | 97.34% |
| Fel d 4 mutant 6 | 96.73% |
| Fel d 4 mutant 7 | 99.26% |
| Fel d 4 mutant 8 | 97.83% |
| Fel d 4 mutant 9 | 99.64% |
| Fel d 4 mutant 10 | 96.87% |
| Fel d 4 mutant 11 | 97.23% |
| Fel d 4 mutant 12 | 98.65% |
| Fel d 4 mutant 13 | 99.16% |
| Fel d 4 mutant 14 | 97.89% |
| Fel d 4 mutant 15 | 99.30% |

### Example 10: Primary Structure Analysis and Molecular Weight Determination of Fel d 4 and Its Mutants

LC-MS molecular weight analysis can accurately reflect whether the primary sequence of a biological macromolecule is correct, including whether the N- and C-terminal sequences are intact, and whether post-translational modifications such as glycosylation, oxidation, and deamidation are present. It is one of the most important analytical methods for biological macromolecules.

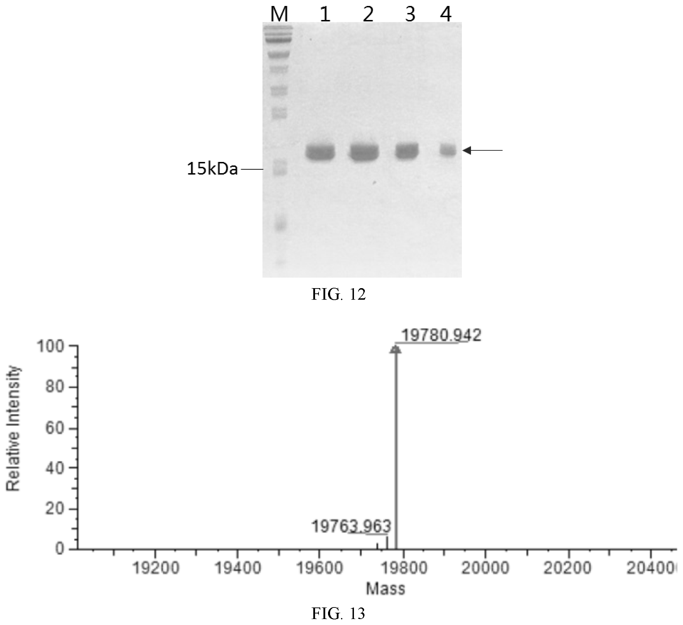

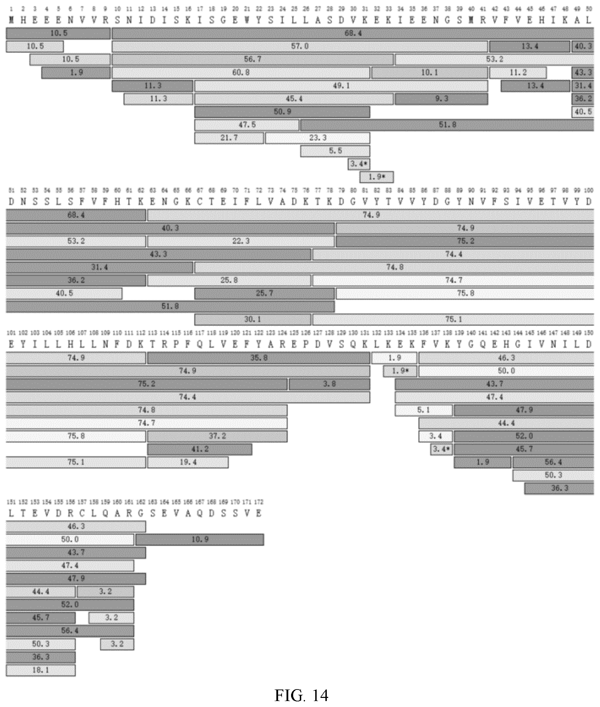

The purified wild-type Fel d 4 and its mutant recombinant proteins were subjected to LC-MS molecular weight determination and primary structure analysis using a Thermo Scientific^{™} high-resolution mass spectrometry system. The results are shown in FIG. 13-FIG. 15 and Table 11. Recombinant proteins expressed in Escherichia coli typically contain an additional N-terminal formylmethionine residue, encoded by the initiation codon, in addition to the native amino acid sequence. In the specific embodiments of the present invention, recombinant Fel d 4 (rFel d 4) was prepared using an Escherichia coli expression system. Therefore, compared with the mature peptide sequence of native Fel d 4 (nFel d 4), as shown in FIG. 14, rFel d 4 contains one additional amino acid residue at the N-terminus, namely formylmethionine, while the remaining primary structure is identical to that of nFel d 4. In addition, the mature peptide of nFel d 4 theoretically forms a disulfide bond between C66 and C156. Due to the presence of an additional N-terminal M in rFel d 4 expressed in Escherichia coli, the disulfide bond is formed between C67 and C157, which is consistent with the theoretical expectation.

As shown by the analytical results, the molecular weight of wild-type Fel d 4 is heterogeneous, exhibiting multiple components with similar molecular weights. In contrast, after mutation at amino acid position 139, all mutants of the present invention exhibit significantly improved molecular weight uniformity. These results further demonstrate that the Fel d 4 mutants of the present invention have advantages in terms of recombinant protein purity and homogeneity, thereby facilitating the correct preparation of Fel d 4 and improving the quality controllability of recombinant Fel d 4 production.

**Table 11**

| **Protein Name** | **Molecular Weight** | **Disulfide Bond Pairing** |
|---|---|---|
| nFel d 4 | Heterogeneous; molecular weight ranges from 19000 to 21000 with multiple components; the proportion of the target molecular weight is less than 5%. | Consistent with theory |
| Fel d 4 mutant 1 | Homogeneous; the proportion of the target molecular weight exceeds 90%. | Consistent with theory |
| Fel d 4 mutant 2 | Homogeneous; the proportion of the target molecular weight exceeds 90%. | Consistent with theory |
| Fel d 4 mutant 3 | Homogeneous; the proportion of the target molecular weight exceeds 90%. | Consistent with theory |
| Fel d 4 mutant 4 | Homogeneous; the proportion of the target molecular weight exceeds 90%. | Consistent with theory |
| Fel d 4 mutant 5 | Homogeneous; the proportion of the target molecular weight exceeds 90%. | Consistent with theory |
| Fel d 4 mutant 6 | Homogeneous; the proportion of the target molecular weight exceeds 90%. | Consistent with theory |
| Fel d 4 mutant 7 | Homogeneous; the proportion of the target molecular weight exceeds 90%. | Consistent with theory |
| Fel d 4 mutant 8 | Homogeneous; the proportion of the target molecular weight exceeds 90%. | Consistent with theory |
| Fel d 4 mutant 9 | Homogeneous; the proportion of the target molecular weight exceeds 90%. | Consistent with theory |
| Fel d 4 mutant 10 | Homogeneous; the proportion of the target molecular weight exceeds 90%. | Consistent with theory |
| Fel d 4 mutant 11 | Homogeneous; the proportion of the target molecular weight exceeds 90%. | Consistent with theory |
| Fel d 4 mutant 12 | Homogeneous; the proportion of the target molecular weight exceeds 90%. | Consistent with theory |
| Fel d 4 mutant 13 | Homogeneous; the proportion of the target molecular weight exceeds 90%. | Consistent with theory |
| Fel d 4 mutant 14 | Homogeneous; the proportion of the target molecular weight exceeds 90%. | Consistent with theory |
| Fel d 4 mutant 15 | Homogeneous; the proportion of the target molecular weight exceeds 90%. | Consistent with theory |

### Example 11: Activity Assay of Fel d 4 Mutants

In this example, the reactivity of recombinant Fel d 4 mutants prepared in the present invention was evaluated using clinical cat allergy sera collected from Jiangsu Provincial People's Hospital between March and May 2023. The assay procedure was as follows:
1. Coating: recombinant Fel d 4 proteins were diluted to 1 µg/mL using 0.05 M carbonate coating buffer (pH 9.6); the diluted proteins were added to a microplate at 100 µL per well and incubated at 2-8°C overnight;
2. Blocking: on the following day, the microplate was washed once with PBST; blocking was performed by adding 2% BSA in PBST at 200 µL per well, followed by incubation at 37°C for 2 h;
3. Sample Preparation: clinical cat allergy positive and negative sera were diluted with 2% BSA in PBST (dilution factors are shown in Table 12); 2% BSA in PBST was used as the blank background control; after removal of liquid from the wells, 100 µL of each diluted sample was added per well and incubated at 37°C with shaking at 300 rpm for 2 h;
4. Detection: the plate was washed three times with PBST; mouse anti-human IgE-HRP secondary antibody diluted at 1:1500 was added at 100 µL per well, followed by incubation at 37°C with shaking at 300 rpm for 1 h;
5. Color Development, Termination, and Reading: the plate was washed three times with PBST; TMB substrate solution (Type VII) was added at 100 µL per well for color development; after incubation at 37°C for 15 min, 50 µL of 2 M sulfuric acid was added to each well to terminate the reaction; the microplate was immediately read at 450 nm using a microplate reader (Thermo Multiskan GO); The readings reflect the binding level between Fel d 4 and specific IgE in the serum.

The results in Table 12 show the reactivity of Fel d 4 mutant 2 and mutant 3 recombinant proteins with different clinical cat allergy sera (microplate reader readings). The results indicate that most of the 11 positive sera exhibited reactivity with both mutant recombinant proteins. The two mutants showed comparable reactivity across different cat allergy positive sera. Serum samples SZ20, 1363, and SZ28 exhibited relatively high reactivity with both mutant recombinant proteins, whereas most samples showed relatively low reactivity. Some results (e.g., SZ19) showed inconsistency between the specific IgE binding level observed in this assay and the corresponding clinical report values, where the clinical values were high but only low levels of specific IgE binding were detected in this example. This suggests that the corresponding patients may not be allergic to Fel d 4 or exhibit low sensitivity to Fel d 4, but are more sensitive to other cat allergens such as Fel d 1. In addition, the inventors of the present application further found that, for the clinical serum samples in Table 12, the reactivity of Fel d 4 mutants was generally lower than that of the type I cat allergen Fel d 1. Only a small number of clinical allergy sera exhibited reactivity to rFel d 4 that was higher than or comparable to that of Fel d 1. It is well known in the art that Fel d 1 is the major cat allergen with a higher sensitization rate, and patients allergic to Fel d 1 are more prevalent in clinical practice.

**Table 12**

| **Clinical Serum** | **Clinical Report Value (kUA/L) (Quantified by Phadia 250)** | **Dilution Factor** | **Microplate Reader Reading** | |
|---|---|---|---|---|
| | | | **mutant2** | **mutant3** |
| 8803 | 1.88 | 10 | 0.0855 | 0.0758 |
| 8779 | 1.19 | 10 | 0.0520 | 0.0452 |
| 4606 | 1.91 | 10 | 0.3649 | 0.3968 |
| 1363 | 4.55 | 10 | 0.7999 | 0.9046 |
| SZ114 | 5.62 | 10 | 0.1568 | 0.0607 |
| SZ15 | 5.62 | 10 | 0.0643 | 0.0585 |
| 8773 | 11.7 | 50 | 0.0555 | 0.0458 |
| CZ224 | 13.2 | 50 | 0.0684 | 0.0439 |
| SZ11 | 16.2 | 50 | 0.1728 | 0.1128 |
| SZ28 | 20.1 | 50 | 0.4664 | 0.5212 |
| SZ19 | 60.5 | 50 | 0.0910 | 0.0629 |
| SZ20 | 64.8 | 50 | 2.0412 | 2.4212 |
| Negative Serum | 0 | 10 | 0.0746 | 0.0693 |

### Example 12: Evaluation of In Vivo Sensitization and Desensitization Efficacy of Fel d 4 Mutants

### 1. Materials

Female BALB/c mice, aged 6-7 weeks at the time of receipt, were obtained from Changzhou Cavens Laboratory Animal Co., Ltd.

Recombinant Fel d 4 mutant solution prepared in the present invention (mutant 2 as an example).

Aluminum hydroxide adjuvant, provided by Thermo Fisher Scientific.

### 2. Preparation of Allergen Samples

Sensitization sample: a Fel d 4 mutant solution at a concentration of 0.0067 mg/mL was mixed with aluminum hydroxide adjuvant at a volume ratio of 3:1 to prepare the sensitization sample. The mixture was thoroughly homogenized prior to injection.

Challenge sample: a Fel d 4 mutant solution at a concentration of 0.05 mg/mL was used as the challenge sample.

### 3. Administration Regimen

### 3.1 Sensitization Phase

Sixteen mice were randomly divided into two groups (n = 8 per group) according to body weight. Grouping and dosing details are shown in Table 13. The Fel d 4 mutant group was subcutaneously injected with the sensitization sample at 1 µg per mouse per dose. The negative control group received an equal volume of blank sample. Administration was performed once weekly for a total of three doses.

**Table 13 Grouping and Administration**

| Group Name | Model-Inducing Agent | Sensitization Dose (µg/mouse/dose) | Challenge Dose (µg/mouse/dose) |
|---|---|---|---|
| Negative Control Group | PBS | 0 | 0 |
| Fel d 4 Mutant Group | mutant 2 | 1 | 2 |

### 3.2 Challenge Phase

One week after the final sensitization, mice in the mutant group were intranasally challenged with the challenge sample at 40 µL per mouse per dose, once daily for 7 consecutive days. The negative control group received an equal volume of blank sample. Details are shown in Table 13.

### 4. Evaluation Parameters

### 4.1 Serum Antibodies

48 hours after the final challenge, whole blood was collected via orbital bleeding. After standing at room temperature for 2-3 h, the collected whole blood samples were centrifuged at 3500 rpm for 10 min to obtain serum. Serum antibodies were measured by ELISA, using Fel d 4 mutant 2 as the coating antigen.

### 4.2 WBC Counts in BALF

Bronchoalveolar lavage fluid (BALF) was collected, and three lavage fractions were pooled and centrifuged. The supernatant was discarded, and the cell pellet was resuspended in 1×PBS buffer. After thorough mixing, cells were stained according to the instructions of the Wright-Giemsa staining kit. Under an upright microscope, stained white blood cells were observed and counted at 200× magnification.

### 4.3 Cytokines in Supernatants of In Vitro-Stimulated Splenocytes

Splenocytes were isolated from mice in each group under sterile conditions and stimulated with the challenge sample. After co-culture with the challenge sample for 144 h, the supernatants were collected. The levels of IL-4, IL-5, IL-10, and IL-13 in the supernatants of in vitro-stimulated splenocytes were measured using a cytometric bead array (CBA) assay.

### 5. Results

### 5.1 Serum Antibodies

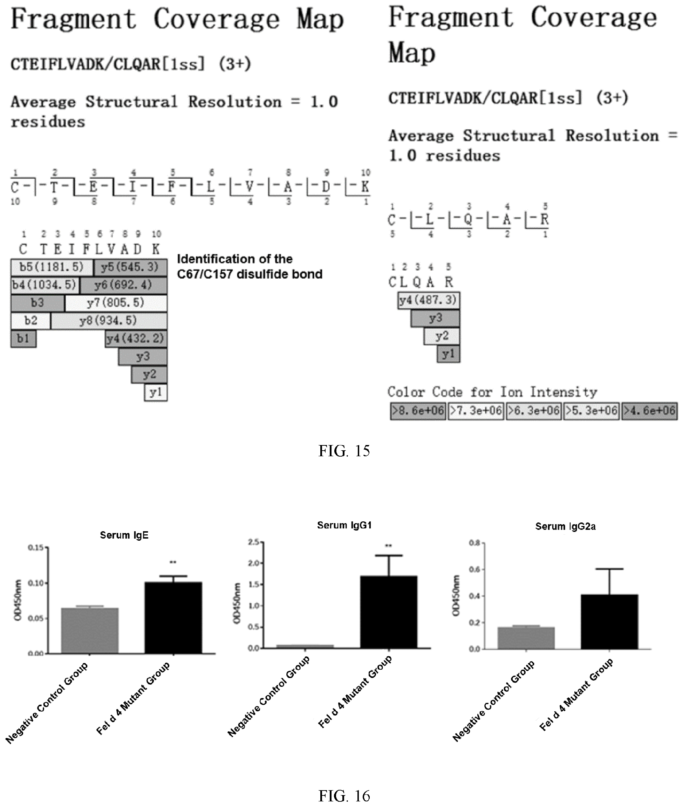

As shown in Table 14 and FIG. 16, the serum antibody assay results of each group indicate that,
IgE levels in the Fel d 4 mutant group were significantly higher than those in the negative control group (p < 0.01).

IgG1 levels in the Fel d 4 mutant group were significantly higher than those in the negative control group (p < 0.01).

IgG2a levels in the Fel d 4 mutant group showed an increasing trend compared with the negative control group.

**Table 14 Serum Antibody Levels (Mean ± SEM)**

| Group Name | Number of Samples (n) | IgE OD Value | IgG1 OD Value | IgG2a OD Value |
|---|---|---|---|---|
| Negative Control Group | 8 | 0.0641±0.0035 | 0.0655±0.0041 | 0.1622±0.0147 |
| Fel d 4 mutant group | 8 | 0.1006±0.0093^{**} | 1.691±0.4927^{**} | 0.4084±0.1976 |

| | | | | |
|---|---|---|---|---|
| Note: ** indicates p < 0.01 compared with the negative control group. | | | | |

### 5.2 WBC Counts in BALF

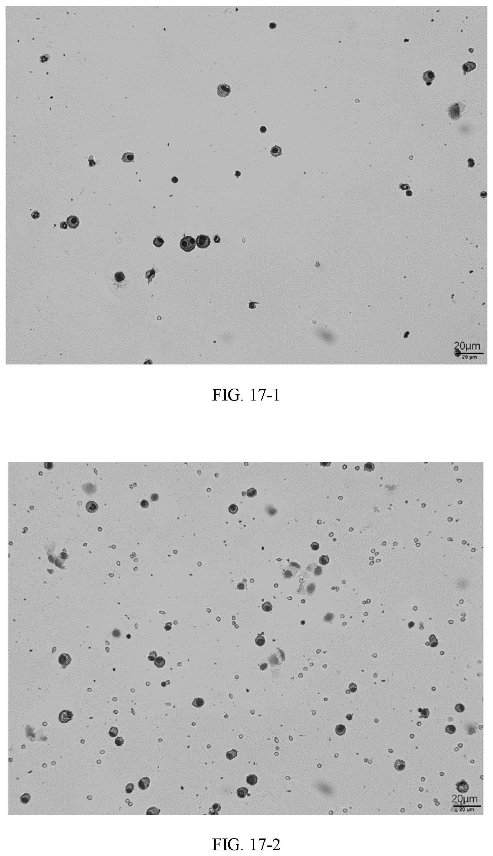

As shown in FIG. 17-1 and FIG. 17-2, microscopic observation of stained BALF cells indicated that white blood cell numbers were markedly increased in the Fel d 4 mutant group compared with the negative control group.

### 5.3 Cytokines in Supernatants of In Vitro-Stimulated Splenocytes

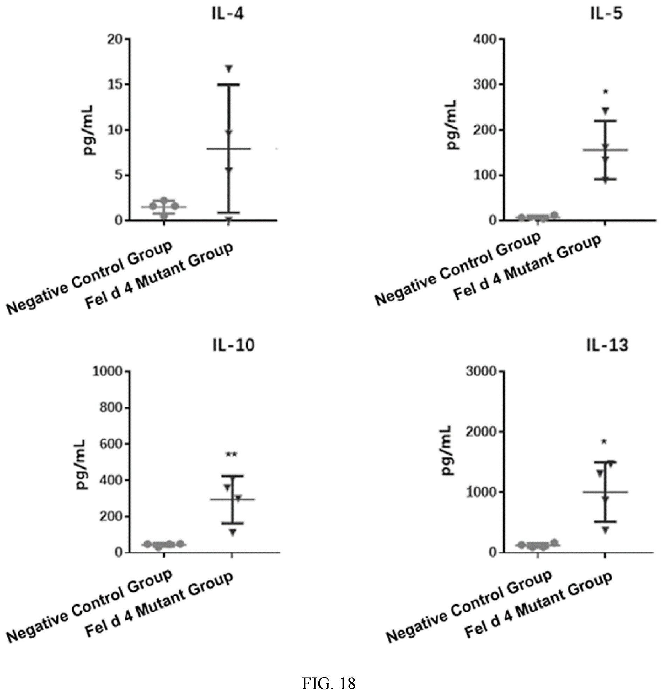

As shown in Table 15 and FIG. 18, Th2-type cytokines (IL-4, IL-5, IL-10, and IL-13) were markedly elevated in the Fel d 4 mutant group. IL-5, IL-10, and IL-13 were significant increased compared with the negative control group (p < 0.05, p < 0.01), while IL-4 showed an increasing trend compared with the negative control group.

**Table 15 TH2-Type Cytokines in Supernatants of In Vitro-Stimulated Splenocytes (Mean ± SEM)**

| Group Name | Number of Samples (n) | Level (pg/mL) | | | |
|---|---|---|---|---|---|
| | | IL-4 | IL-5 | IL-10 | IL-13 |
| Negative Control Group | 4 | 1.49±0.35 | 6.97±1.71 | 43.8±4.47 | 120.74±17.77 |
| Fel d 4 mutant group | 4 | 7.95±3.53 | 156.28±32.21* | 294.24±65.15** | 1005.85±246.75* |

| | | | | | |
|---|---|---|---|---|---|
| Note: * indicates p < 0.05, ** indicates p < 0.01 compared with the negative control group | | | | | |

### 6. Conclusion

The results of the present study demonstrate that, in an allergic airway inflammation model established by sensitization and challenge with the Fel d 4 mutant protein of the present invention, mice in the Fel d 4 mutant group exhibited elevated levels across multiple parameters, including antibody responses, target organ inflammation, and cytokine production. These findings indicate that the Fel d 4 mutant protein of the present invention possesses immunogenicity in vivo, is capable of inducing allergic responses, increasing allergen-specific antibody levels, and stimulating immune responses. Accordingly, the Fel d 4 mutant protein of the present invention has potential for use as an active ingredient in cat allergen immunotherapy or as a component of diagnostic reagents for cat allergy diagnosis, thereby enabling application in the diagnosis and treatment of cat allergic diseases.

### Example 13: Design of Fel d 7 Mutants and Construction of Expression Strains

Native Fel d 7 may exist as multiple isoallergens or variants. In this example, the nFel d 7 sequence with GenBank accession No. ADK56160 is used as a representative.

The mature peptide sequence of nFel d 7 was obtained according to the GenBank accession and is shown in SEQ ID NO: 10. The corresponding DNA sequence was obtained by reverse translation and was codon-optimized for an Escherichia coli expression system by Nanjing GenScript Biotechnology Co., Ltd., followed by synthesis to obtain the coding DNA sequence of nFel d 7, as shown in SEQ ID NO: 11. A deletion or amino acid substitution mutation at position 101 of nFel d 7 was introduced by PCR (mutation information is shown in Table 16). NdeI and XhoI restriction sites were introduced at the 5' end and 3' end of the mutant coding sequences, respectively. Taking Mutant 1 as an example, its DNA sequence is shown in SEQ ID NO: 12.

Recombinant expression of Fel d 7 according to the present invention may be carried out using expression vectors based on a T7 promoter, such as pET32a, pET26b, pET28a, and pDEST14, or expression vectors based on a temperature-inducible PL-PR promoter, such as pBV220. When using expression vectors based on the PL-PR promoter, any genetically engineered Escherichia coli strain may be selected, including but not limited to BL21 (DE3), BL21 AI (DE3), Top10, DH5α, JM109, Rosetta (DE3), Rosetta gamiB (DE3), and BL21 (DE3) pLys. When using expression vectors based on the T7 promoter, suitable Escherichia coli strains include BL21 (DE3), BL21 AI (DE3), Rosetta (DE3), Rosetta gamiB (DE3), and BL21 (DE3) pLys. In this example, the pET26b vector and BL21 (DE3) host strain were used for illustration.

The DNA sequences of the above Fel d 7 mutants were cloned into the pET26b vector to construct pET26b-Fel d 7 mutant expression plasmids, which were then transformed into the BL21 (DE3) host strain to obtain BL21 (DE3)-pET26b-Fel d 7 mutant expression strains.

**Table 16 Summary of Mutation Information for Each Sample**

| **Protein Name** | **Mutation at position 101** |
|---|---|
| nFel d 7 | No mutation |
| Fel d 7 mutant 1 | Deletion of the original C at position 101 |
| Fel d 7mutant2 | C101G |
| Fel d 7mutant3 | C101A |
| Fel d 7mutant4 | C101V |
| Fel d 7mutant5 | C101L |
| Fel d 7mutant6 | C101I |
| Fel d 7mutant7 | C101Y |
| Fel d 7mutant8 | C101S |
| Fel d 7mutant9 | C101R |
| Fel d 7mutant10 | C101H |
| Fel d 7mutant11 | C101F |
| Fel d 7mutant12 | C101W |
| Fel d 7mutant13 | C101M |
| Fel d 7mutant14 | C101T |
| Fel d 7mutant15 | C101K |

### Example 14: Recombinant Expression of Fel d 7 and Its Mutants

Expression of Fel d 7 mutant 1 in the pET26b vector and BL21 (DE3) host strain is described below. The same method was used for the expression of nFel d 7 and other mutants.
1. BL21 (DE3)-pET26b-Fel d 7 mutant 1 strain was streaked from a glycerol stock onto an LB agar plate containing 50 µg/mL and incubated at 37°C for 12 h;
2. A single colony was picked from the agar plate and inoculated into 10 mL of TB liquid medium, and cultured at 37°C with shaking at 220 rpm for 8 h; the resulting culture was used as the primary seed culture;
3. The primary seed culture was inoculated at a ratio of 5% into ten flasks each containing 500 mL of TB liquid medium, followed by incubation at 37°C with shaking at 220 rpm; when the OD reached 0.8, expression was induced by adding 0.5 mM IPTG; after induction for 4 h, the cells were harvested by centrifugation for subsequent use.

### Example 15: Extraction and Purification of Fel d 7 and Its Mutants

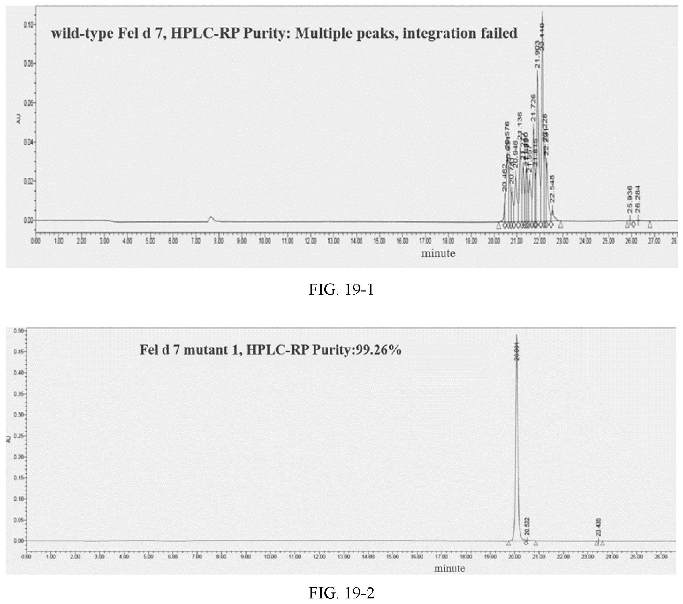

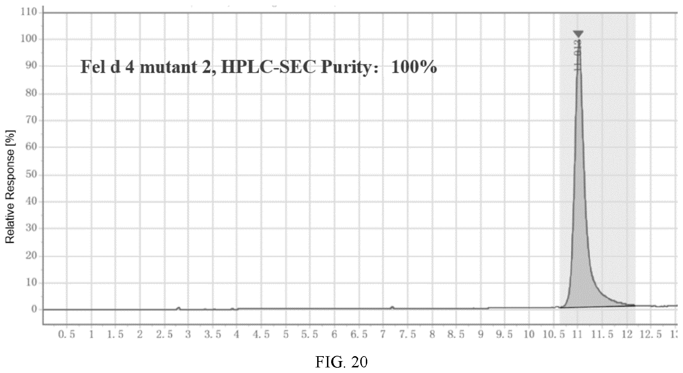

Extraction and purification of Fel d 7 mutant 1 are described below. The same process was used to obtain nFel d 7 and other mutants.
1. Fermented cells were resuspended in 10 mM PB buffer (pH 7.4) to a concentration of 80 g/L and uniformly dispersed; cell disruption was performed using a high-pressure homogenizer at 900 ± 50 bar for three cycles, with the sample temperature maintained below 25°C throughout the process; after disruption, the homogenate was centrifuged, and the supernatant was collected;
2. The supernatant was filtered through a 0.45 µm membrane; the resulting filtrate was concentrated using an ultrafiltration membrane with a molecular weight cutoff of 100 kDa, followed by concentration and buffer exchange with 10 mM PB (pH 8.0), and the filtrate was collected;
3. First-step purification: the filtrate was purified using Q Sepharose Fast Flow; a linear gradient of NaCl was applied to reach 0.5 M over 10 column volumes, and the target protein peak was collected; the binding buffer was 10 mM PB (pH 8.0), and the elution buffer was 10 mM PB + 1 M NaCl (pH 8.0);
4. Second-step purification: ammonium sulfate was added to the sample from the first-step purification to a final concentration of 2 M, and the pH was adjusted to 7.4; purification was performed using a Phenyl Sepharose High Performance 6 (HS) column; a linear gradient was applied over 10 column volumes, reaching 100% elution buffer, and the target protein peak was collected; the elution buffer was 10 mM PB (pH 7.4), and the binding buffer was 10 mM PB + 2 M ammonium sulfate (pH 7.4);
5. The target protein peaks collected from the two chromatographic steps were subjected to ultrafiltration and buffer exchange into 20 mM PB (pH 7.0); after sterile filtration, the target protein bulk solution was obtained;
6. The final samples were analyzed by HPLC-SEC, HPLC-RP, and non-reducing SDS-PAGE, and the results are shown in FIG. 19-FIG. 21.

The results showed that both wild-type Fel d 7 and mutant 1 could achieve equivalent electrophoretic purity under the same process conditions, and no visible impurities were observed in non-reducing SDS-PAGE. However, the HPLC-RP profile of wild-type Fel d 7 exhibited a distinct impurity peak that was difficult to separate, whereas this phenomenon was not observed in the mutants. It was inferred that the purified wild-type protein contained a related protein species with a molecular weight similar to that of the target protein but with significantly different hydrophobic properties.

Further analysis of HPLC-RP purity for each mutant (as shown in Table 17) revealed that all mutants achieved a purity of over 95% under the same process conditions. Since all mutants contain mutations at position 101 of nFel d 7, mutation at this position is considered a key factor in improving the RP purity of Fel d 7. Compared with wild-type Fel d 7, the Fel d 7 mutants of the present invention exhibit superior purity and homogeneity of the recombinant protein, and are more advantageous for recombinant production.

**Table 17 Summary of HPLC- RP Purity of Each Sample**

| **Protein Name** | **HPLC-RPPurity** |
|---|---|
| nFel d 7 | Integration failed |
| Fel d 7 mutant 1 | 99.26% |
| Fel d 7 mutant 2 | 97.82% |
| Fel d 7 mutant 3 | 98.23% |
| Fel d 7 mutant 4 | 99.35% |
| Fel d 7 mutant 5 | 96.94% |
| Fel d 7 mutant 6 | 98.61% |
| Fel d 7 mutant 7 | 95.98% |
| Fel d 7 mutant 8 | 97.32% |
| Fel d 7 mutant 9 | 98.43% |
| Fel d 7 mutant 10 | 97.42% |
| Fel d 7 mutant 11 | 97.89% |
| Fel d 7 mutant 12 | 99.15% |
| Fel d 7 mutant 13 | 96.94% |
| Fel d 7 mutant 14 | 97.13% |
| Fel d 7 mutant 15 | 98.52% |

### Example 16: Primary Structure Analysis and Molecular Weight Determination of Fel d 7 and Its Mutants

LC-MS molecular weight analysis can accurately reflect whether the primary sequence of a biological macromolecule is correct, including whether the N- and C-terminal sequences are intact, and whether post-translational modifications such as glycosylation, oxidation, and deamidation are present. It is one of the most important analytical methods for biological macromolecules.

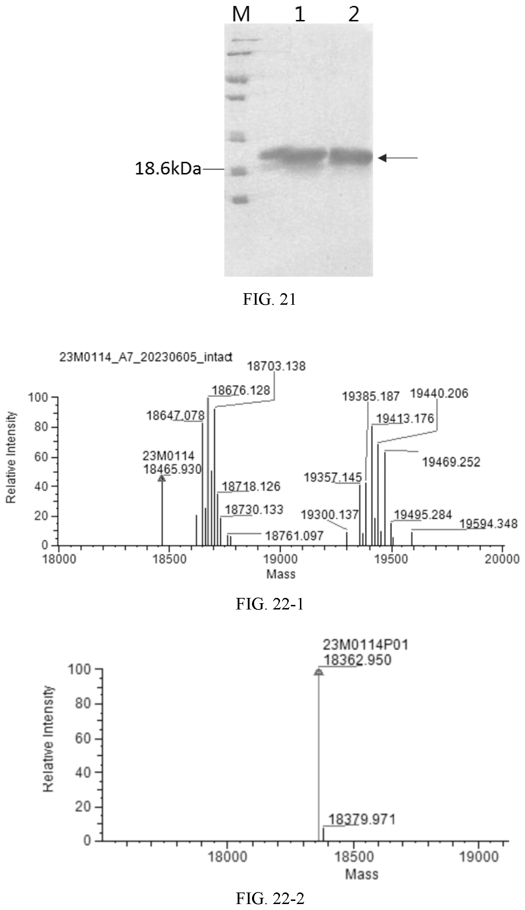

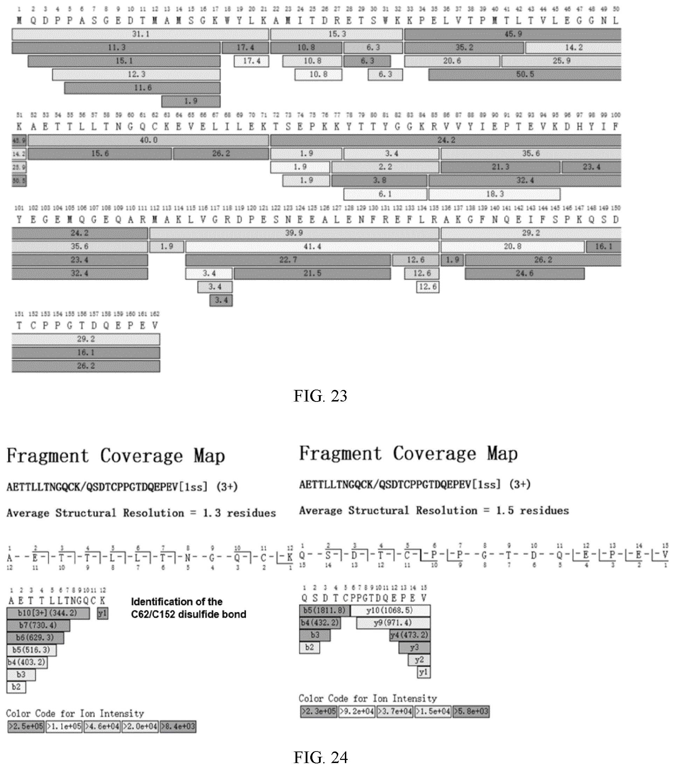

The purified wild-type Fel d 7 and its mutant recombinant proteins were subjected to LC-MS molecular weight determination and primary structure analysis using a Thermo Scientific^{™} high-resolution mass spectrometry system. The results are shown in FIG. 22-FIG. 24 and Table 18. Recombinant proteins expressed in Escherichia coli typically contain an additional N-terminal formylmethionine residue, encoded by the initiation codon, in addition to the native amino acid sequence. In the specific embodiments of the present invention, recombinant Fel d 7 (rFel d 7) was prepared using an Escherichia coli expression system. Therefore, compared with the mature peptide sequence of native Fel d 7 (nFel d 7), as shown in FIG. 23, rFel d 7 contains one additional amino acid residue at the N-terminus, namely formylmethionine, while the remaining primary structure is identical to that of nFel d 7. In addition, the mature peptide of nFel d 7 theoretically forms a disulfide bond between C62 and C152. Since the rFel d 7 expressed in Escherichia coli according to the present invention contains an additional N-terminal M and a deletion at C101 relative to nFel d 7, the detected disulfide bond formed between C62 and C152 is consistent with the theoretical expectation.

As shown by the analytical results, the molecular weight of wild-type Fel d 7 is heterogeneous, exhibiting multiple components with similar molecular weights. In contrast, after mutation at amino acid position 101, all mutants of the present invention exhibit significantly improved molecular weight uniformity. These results further demonstrate that the Fel d 7 mutants of the present invention have advantages in terms of recombinant protein purity and homogeneity, thereby facilitating the correct preparation of Fel d 7 and improving the quality controllability of recombinant Fel d 7 production.

**Table 18 Summary of LC-MS Analysis Results for Each Sample**

| **Protein Name** | **Molecular Weight** | **Disulfide Bond Pairing** |
|---|---|---|
| nFel d 7 | Heterogeneous; molecular weight ranges from 18000 to 20000 with multiple components; the proportion of the target molecular weight is less than 20%. | Consistent with theory |
| Fel d 7 mutant 1 | Homogeneous; the proportion of the target molecular weight exceeds 90%. | Consistent with theory |
| Fel d 7 mutant 2 | Homogeneous; the proportion of the target molecular weight exceeds 90%. | Consistent with theory |
| Fel d 7 mutant 3 | Homogeneous; the proportion of the target molecular weight exceeds 90%. | Consistent with theory |
| Fel d 7 mutant 4 | Homogeneous; the proportion of the target molecular weight exceeds 90%. | Consistent with theory |
| Fel d 7 mutant 5 | Homogeneous; the proportion of the target molecular weight exceeds 90%. | Consistent with theory |
| Fel d 7 mutant 6 | Homogeneous; the proportion of the target molecular weight exceeds 90%. | Consistent with theory |
| Fel d 7 mutant 7 | Homogeneous; the proportion of the target molecular weight exceeds 90%. | Consistent with theory |
| Fel d 7 mutant 8 | Homogeneous; the proportion of the target molecular weight exceeds 90%. | Consistent with theory |
| Fel d 7 mutant 9 | Homogeneous; the proportion of the target molecular weight exceeds 90%. | Consistent with theory |
| Fel d 7 mutant 10 | Homogeneous; the proportion of the target molecular weight exceeds 90%. | Consistent with theory |
| Fel d 7 mutant 11 | Homogeneous; the proportion of the target molecular weight exceeds 90%. | Consistent with theory |
| Fel d 7 mutant 12 | Homogeneous; the proportion of the target molecular weight exceeds 90%. | Consistent with theory |
| Fel d 7 mutant 13 | Homogeneous; the proportion of the target molecular weight exceeds 90%. | Consistent with theory |
| Fel d 7 mutant 14 | Homogeneous; the proportion of the target molecular weight exceeds 90%. | Consistent with theory |
| Fel d 7 mutant 15 | Homogeneous; the proportion of the target molecular weight exceeds 90%. | Consistent with theory |

### Example 17: Evaluation of In Vivo Sensitization and Desensitization Efficacy of Fel d 7 Mutants

### 1. Materials

Female BALB/c mice, aged 6-7 weeks at the time of receipt, were obtained from Changzhou Cavens Laboratory Animal Co., Ltd.

Recombinant Fel d 7 mutant solution prepared in the present invention (mutant 1 as an example).

Aluminum hydroxide adjuvant, provided by Thermo Fisher Scientific.

### 2. Preparation of Allergen Samples

Sensitization sample: a Fel d 7 mutant solution at a concentration of 0.0067 mg/mL was mixed with aluminum hydroxide adjuvant at a volume ratio of 3:1 to prepare the sensitization sample. The mixture was thoroughly homogenized prior to injection.

Challenge sample: a Fel d 7 mutant solution at a concentration of 0.05 mg/mL was used as the challenge sample.

### 3. Administration Regimen

### 3.1 Sensitization Phase

Sixteen mice were randomly divided into two groups (n = 8 per group) according to body weight. Grouping and dosing details are shown in Table 19. The Fel d 7 mutant group was subcutaneously injected with the sensitization sample at 1 µg per mouse per dose. The negative control group received an equal volume of blank sample. Administration was performed once weekly for a total of three doses.

**Table 19 Grouping and Administration**

| Group Name | Model-Inducing Agent | Sensitization Dose (µg/mouse/dose) | Challenge Dose (µg/ mouse /dose) |
|---|---|---|---|
| Negative Control Group | PBS | 0 | 0 |
| Fel d 7 Mutant Group | Mutant 1 | 1 | 2 |

### 3.2 Challenge Phase

One week after the final sensitization, mice in the mutant group were intranasally challenged with the challenge sample at 40 µL per mouse per dose, once daily for 7 consecutive days. The negative control group received an equal volume of blank sample. Details are shown in Table 19.

### 4. Evaluation Parameters

### 4.1 Penh

24 hours after the final challenge, Penh values of all mice were measured using a whole-body plethysmography (WBP) system at methacholine (Mch) concentrations of 0, 6.25, 12.5, 25, and 50 mg/mL. Penh values reflect airway resistance, and higher Penh values indicate higher airway responsiveness and more severe airway symptoms.

### 4.2 Serum Allergen-Specific IgE Antibodies

48 hours after the final challenge, whole blood was collected via orbital bleeding. After standing at room temperature for 2-3 h, the collected whole blood samples were centrifuged at 3500 rpm for 10 min to obtain serum. Serum antibodies were measured by ELISA using mutant 1 as the coating antigen.

### 4.3 WBC Counts in BALF

Bronchoalveolar lavage fluid (BALF) was collected, and three lavage fractions were pooled and centrifuged. The supernatant was discarded, and the cell pellet was resuspended in 1×PBS buffer. After thorough mixing, cells were stained according to the instructions of the Wright-Giemsa staining kit. Under an upright microscope, stained white blood cells were observed and counted at 200× magnification.

### 4.4 Cytokines in Supernatants of In Vitro-Stimulated Splenocytes

Splenocytes were isolated from mice in each group under sterile conditions and stimulated with the challenge sample. After co-culture with the challenge sample for 144 h, the supernatants were collected. The levels of IL-4, IL-5, IL-10, and IL-13 in the supernatants of in vitro-stimulated splenocytes were measured using a cytometric bead array (CBA) assay.

### 5. Results

### 5.1 Penh

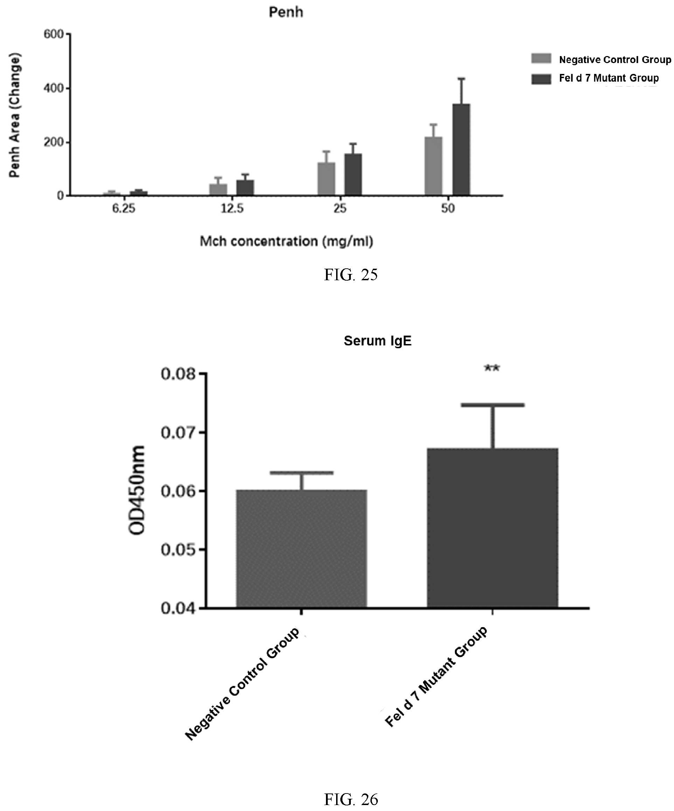

As shown in Table 20 and FIG. 25, Penh values in the Fel d 7 mutant group were higher than those in the negative control group at different MCh challenge concentrations.

**Table 20 Mouse Penh Values (Mean ± SEM)**

| Group Name | Number of Samples (n) | Penh | | | |
|---|---|---|---|---|---|
| | | Mch 6.25mg/mL | Mch 12.5mg/mL | Mch 25mg/mL | Mch 50mg/mL |
| Negative Control Group | 8 | 7.78±10.05 | 40.57±27.84 | 120.64±45.19 | 216.37±49.33 |
| Fel d 7 Mutant Group | 8 | 14.55±7.67 | 56.55±23.48 | 154.08±40.86 | 339.31±96.07 |

### 5.2 Serum Allergen-Specific IgE Antibodies

As shown in Table 21 and FIG. 26, the serum allergen-specific IgE antibody results of each group indicate that, IgE levels in the Fel d 7 mutant group were significantly higher than those in the negative control group (p < 0.01).

**Table 21 Serum Antibody Levels (Mean ± SEM)**

| Group Name | Number of Samples (n) | IgE OD Value |
|---|---|---|
| Negative Control Group | 8 | 0.06±0.0032 |
| Fel d 7 Mutant Group | 8 | 0.0672±0.0076^{**} |

| | | |
|---|---|---|
| Note: ** indicates p < 0.01 compared with the negative control group (mutant 1). | | |

### 5.3 WBC Counts in BALF

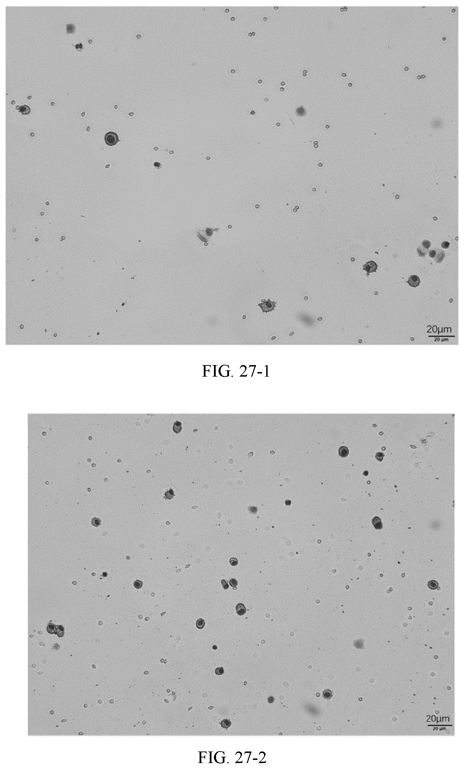

As shown in FIG. 27-1 and FIG. 27-2, microscopic observation of stained BALF cells indicated that white blood cell numbers were markedly increased in the Fel d 7 mutant group compared with the negative control group.

### 5.4 Cytokines in Supernatants of In Vitro-Stimulated Splenocytes

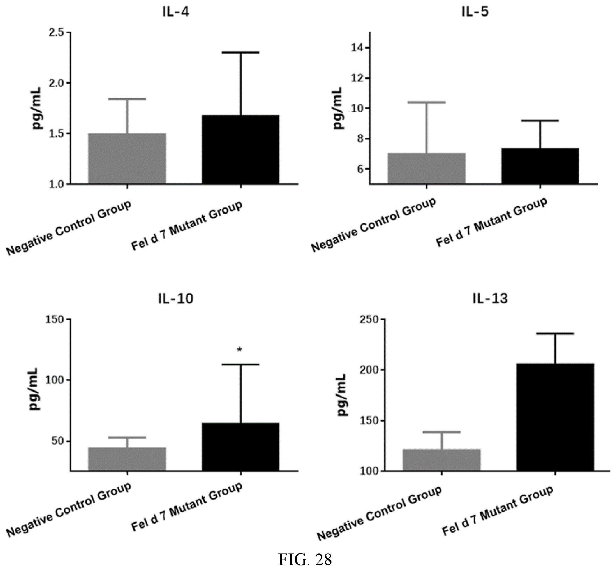

As shown in Table 22 and FIG. 28, Th2-type cytokines (IL-4, IL-5, IL-10, and IL-13) showed an increasing trend in the Fel d 7 mutant group. IL-10 was significantly increased compared with the negative control group (p < 0.05). After adjustment of the sensitization and challenge doses of rFel d 7, the levels of IL-4, IL-5, and IL-13 in the supernatants of in vitro-stimulated splenocytes from the Fel d 7 mutant group were also significantly higher than those in the negative control group.

**Table 22 TH2-Type Cytokines in Supernatants of In Vitro-Stimulated Splenocytes (Mean ± SEM)**

| Group Name | Number of Samples (n) | Level (pg/mL) | | | |
|---|---|---|---|---|---|
| | | IL-4 | IL-5 | IL-10 | IL-13 |
| Negative Control Group | 4 | 1.49±0.35 | 6.97±1.71 | 43.8±4.47 | 120.74±17.77 |
| Fel d 7 Mutant Group | 4 | 1.67±0.63 | 7.31±0.94 | 64.32±24.29* | 205.63±30.29 |

| | | | | | |
|---|---|---|---|---|---|
| Note: * indicates p < 0.05 compared with the negative control group | | | | | |

### 6. Conclusion

The results of the present study demonstrate that, in an allergic airway inflammation model established by sensitization and challenge with the Fel d 7 mutant protein of the present invention, mice in the Fel d 7 mutant group exhibited elevated levels across multiple parameters, including airway symptoms, antibody responses, target organ inflammation, and cytokine production. These parameters were further elevated at higher sensitization and challenge doses. These findings indicate that the Fel d 7 mutant protein of the present invention possesses immunogenicity in vivo, is capable of inducing increases in allergen-specific antibodies, triggering allergic responses, and stimulating immune responses. Accordingly, the Fel d 7 mutant protein of the present invention has potential for use as an active ingredient in cat allergen immunotherapy or as a component of diagnostic reagents for cat allergy diagnosis,thereby enabling application in the diagnosis and treatment of cat allergic diseases.

### Example 18: Evaluation of the Effect of a Recombinant Cat Allergen Protein Combination on the Induction of Allergic Airway Inflammation in Mice

### 1. Materials

Female BALB/c mice, aged 8-9 weeks at the time of use, were obtained from Shanghai SLAC Laboratory Animal Co., Ltd.

Cat dander extract with a total protein concentration of 1 mg/mL, batch No. F2124A, was provided by Wolcavi (Beijing) Biotechnology Co., Ltd.

### Aluminum hydroxide adjuvant, batch No. 230017, was provided by SERVA.

### 2. Preparation of Samples

### 2.1 Sensitization Sample

Recombinant Fel d 1, recombinant Fel d 4, and recombinant Fel d 7 were diluted with normal saline for injection (NS) to concentrations of 0.04 mg/mL, respectively (the recombinant allergen proteins were prepared according to the foregoing examples, taking Fel d 1-1, Fel d 4 mutant 2, and Fel d 7 mutant 1 as examples; the same applies hereinafter). Equal volumes of the three solutions were thoroughly mixed to obtain a recombinant protein solution. Separately, the cat dander extract solution was diluted with NS to a concentration of 0.1 mg/mL. Equal volumes of the recombinant protein solution and the cat dander extract solution were thoroughly mixed, followed by dropwise addition of aluminum hydroxide adjuvant at three times the volume. The mixture was thoroughly mixed using a 3D mixer for 30 min. The sensitization sample was freshly prepared before use, shaken well prior to administration, and injected immediately after withdrawal to prevent precipitation.

### 2.2 Challenge Sample

Recombinant Fel d 1, recombinant Fel d 4, and recombinant Fel d 7 were diluted with NS to concentrations of 0.2 mg/mL, respectively. Equal volumes of the three solutions were thoroughly mixed to obtain a recombinant protein solution. Separately, the cat dander extract solution was diluted with NS to a concentration of 0.25 mg/mL. Equal volumes of the recombinant protein solution and the cat dander extract solution were thoroughly mixed to prepare a mixed cat allergen challenge sample solution.

### 2.3 Recombinant Protein Combination

High-dose recombinant protein combination group: 0.7446 mg of recombinant protein mixed powder (recombinant Fel d 1, recombinant Fel d 4, and recombinant Fel d 7 mixed at a mass ratio of 2:1:1) was weighed and dissolved in 621 µL ultrapure water. The mixture was thoroughly mixed using a pipette to obtain a recombinant protein solution at a concentration of 1.2 mg/mL (calculated based on the content of major allergens), which was used for administration in the high-dose recombinant protein combination group.

Medium-dose recombinant protein combination group: 311 µL of the high-dose recombinant protein combination solution at a concentration of 1.2 mg/mL was mixed with 311 µL ultrapure water to obtain a recombinant protein solution at a concentration of 0.6 mg/mL (calculated based on the content of major allergens), which was used for administration in the medium-dose recombinant protein combination group.

Low-dose recombinant protein combination group: 311 µL of the medium-dose recombinant protein combination solution at a concentration of 0.6 mg/mL was mixed with 311 µL ultrapure water to obtain a recombinant protein solution at a concentration of 0.3 mg/mL (calculated based on the content of major allergens), which was used for administration in the low-dose recombinant protein combination group.

### 3 Administration Regimen

### 3.1 Modeling Phase

96 female BALB/c mice were randomly divided into two groups according to body weight: a negative control group and a model control group.

Sensitization: after grouping, mice in the model control group were intraperitoneally injected with 0.2 mL per mouse per dose of the mixed cat allergen sensitization sample solution once weekly for three consecutive weeks. The negative control group received NS in the same manner.

Challenge: seven days after the final sensitization, mice in the model control group were intranasally challenged with the mixed cat allergen challenge sample solution at 40 µL per mouse per dose, once daily for 7 consecutive days. The negative control group received NS in the same manner.

Grouping and administration information are shown in Table 23.

**Table 23 Grouping and Administration Information for Mice of Modeling Period**

| **Group Name** | **Number of Animals** | **Sensitization Dose (µg/mouse/dose)** | **Challenge Dose (µg/mouse/dose)** |
|---|---|---|---|
| Negative Control Group | 12 | 0 | 0 |
| Model Group | 80 | 1+2.5 | 4+5 |

| | | | |
|---|---|---|---|
| Notes: (1) The doses in the table, e.g., "1+2.5", represent 1 µg/mouse/dose of recombinant sample and 2.5 µg/mosue/dose of extract (CD); other cases are omitted for brevity. (2) Animals are tested for specific antibodies in serum, and are screened for enrollment based on the test results. | | | |

### 3.2 Treatment Phase

Among the 80 mice in the model group, 48 mice with relatively high serum levels of specific IgE and IgG1 and in good general condition were selected and randomly divided into four groups: a model control group, a low-dose recombinant combination group, a medium-dose recombinant combination group, and a high-dose recombinant combination group, with 12 mice in each group. The mice in the negative control group were the same as those used in the modeling period, with 12 mice in total. Details are shown in Table 24.

**Table 24 Grouping and administration information**

| Group Name | | Test Article | Administration Dose (µg/mouse/dose) | Number of Mice (n) |
|---|---|---|---|---|
| | | | Calculated based on the content of major allergens | |
| 1 | Negative Control Group | NS | - | 12 |
| 2 | Model Control Group | NS | - | 12 |
| 3 | Low-dose Recombinant combination Group | Recombinant Protein mixed Powder | 6 | 12 |
| 4 | Medium-dose Recombinant combination Group | Recombinant Protein mixed Powder | 12 | 12 |
| 5 | High-dose Recombinant combination Group | Recombinant Protein mixed Powder | 24 | 12 |

Each group received the corresponding test article solution according to Table 24.

Sublingual administration procedure: mice were manually restrained, and 20 µL of the corresponding test article solution shown in Table 24 was accurately pipetted and administered dropwise under the tongue at a constant rate. The mice were continuously restrained for 20 s to prevent immediate swallowing of the test article solution. Administration frequency and duration: 5 times per week for 8 consecutive weeks.

Within 3 days after the final sublingual administration, mice in each group were intranasally challenged with the cat mixed allergen challenge sample solution at 40 µL per mouse per dose, once daily for 7 consecutive days. The negative control group received NS in the same manner.

### 4. Evaluation Parameters

### 4.1 Penh

24 hours after the final challenge, Penh values of all mice were measured using a whole-body plethysmography (WBP) system at methacholine (Mch) concentrations of 0, 6.25, 12.5, 25, and 50 mg/mL.

### 4.2 Serum IgE Levels

48 hours after the final challenge, whole blood was collected via orbital bleeding. After standing at room temperature for 2-3 h, the collected whole blood was centrifuged at 3500 rpm for 10 min to obtain serum. Serum IgE levels were measured by ELISA.

### 4.3 WBC and IgE Levels in BALF

Bronchoalveolar lavage fluid (BALF) was collected, and three lavage fractions were pooled and centrifuged at 800 rpm for 10 min. The supernatant was discarded, and the cell pellet was resuspended in 0.5 mL precooled sterile 1×PBS buffer containing 1% BSA and thoroughly mixed. Eosinophils (EOS) in the BALF suspension were measured using a Siemens ADVIA 2120i hematology analyzer. The remaining BALF suspension was diluted 10-fold with 1×PBS buffer containing 1% BSA, followed by centrifugation at 800 rpm for 5 min to prepare cell smears. Cells were stained according to the instructions of the Wright-Giemsa staining kit. Under an upright microscope at 200× magnification, three random visual fields were selected, and stained cells were counted using ImageJ software to obtain the average value.

A separate aliquot of the supernatant was used to determine IgE levels in BALF by ELISA.

### 4.4 Cytokines in Supernatants of In Vitro-Stimulated Splenocytes

Splenocytes were isolated from mice in each group under sterile conditions and stimulated with the challenge sample. After co-culture with the challenge sample for 144 h, the supernatants were collected. The levels of IL-4, IL-5, IL-10, and IL-13 in the supernatants of in vitro-stimulated splenocytes were measured using a cytometric bead array (CBA) assay.

### 5. Results

### 5.1 Penh

After sublingual administration treatment, Penh values in the model control group were significantly higher than those in the negative control group under stimulation with different concentrations of Mch (P < 0.05, P < 0.01). Penh values in the low-, medium-, and high-dose recombinant protein combination groups were all lower than those in the model control group under stimulation with different concentrations of Mch. At a Mch concentration of 6.25 mg/mL, Penh values in the medium- and high-dose groups were significantly lower than those in the model control group (P < 0.05, P < 0.01). These results indicate that sublingual administration of the recombinant protein combination improved airway hyperresponsiveness in the mouse allergic airway inflammation model.

**Table 25 Mouse Penh Values (Mean ± SEM)**

| **Group Name** | **Number of Samples (n)** | **Penh** | | | |
|---|---|---|---|---|---|
| | | **Mch 6.25mg/mL** | **Mch 12.5mg/mL** | **Mch 25mg/mL** | **Mch 50mg/mL** |
| Negative Control Group | 12 | 17.87±7.71 * | 24.36±6.93 ** | 102.14±22.4 6 ** | 205.37±23.7 1 ** |
| Model Control Group | 12 | 49.97±9.79 | 154.55±35.37 | 288.21±54.3 8 | 641.92±132. 71 |
| Low-dose Recombinant Combination group | 12 | 35.52±7.14 | 123.94±20.45 | 283.79±37.8 4 | 482.24±93.5 2 |
| Medium-dose Recombinant Combination group | 12 | 22.47±7.02 * | 89.38±19.82 | 233.00±26.1 3 | 474.50±105. 60 |
| High-dose Recombinant Combination group | 12 | 18.09±4.43 * * | 76.6±12.34 | 274.92±46.0 5 | 437.38±71.9 7 |

| | | | | | |
|---|---|---|---|---|---|
| Note: * indicates p < 0.05, ** indicates p < 0.01 compared with the model control group. | | | | | |

### 5.2 IgE Levels in Serum and BALF

Serum IgE: the medium- and high-dose recombinant protein combination groups showed lower serum IgE levels than the model control group, and the high-dose group was significantly lower than the model control group (p < 0.05).

BALF IgE: IgE levels in BALF in the low-, medium-, and high-dose recombinant protein combination groups were all lower than those in the model control group, among which the medium- and high-dose groups were significantly lower than the model control group (p < 0.05).

These results indicate that local and systemic allergen-induced immune responses were reduced after treatment with the recombinant protein combination.

**Table 26 Serum Antibody Levels (Mean ± SEM)**

| **Group Name** | **Number of Samples (n)** | **Serum IgE OD Value** | **BALF IgE OD Value** |
|---|---|---|---|
| Negative Control Group | 12 | 0.0553±0.0009 ** | 0.0535±0.001 ** |
| Model Control Group | 12 | 0.3073±0.0385 | 1.0435±0.2199 |
| Low-dose Group | 12 | 0.3331±0.0863 | 0.9707±0.2209 |
| Medium-dose Group | 12 | 0.2282±0.0420 | 0.5816±0.1465* |
| High-dose Group | 12 | 0.2700±0.0346* | 0.7038±0.1086* |

| | | | |
|---|---|---|---|
| Note: * indicates p < 0.05, ** indicates p < 0.01 compared with the model control group. 5.3 WBC and EOS Counts in BALF | | | |

EOS counts in BALF were significantly lower in all recombinant protein combination treatment groups than in the model control group (p < 0.01). WBC count results showed that all recombinant protein combination treatment groups had lower WBC counts than the model control group, among which the high-dose group was significantly lower than the model control group (p < 0.01). These results indicate that pulmonary inflammation in mice was markedly improved after treatment with the recombinant protein combination.

**Table 27 WBC Counts in BALF (Mean ± SEM)**

| **Group Name** | **Number of Samples (n)** | **abs_eos (x10⁹ cells/L)** | **WBC** ( 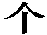 ) |
|---|---|---|---|
| Negative Control Group | 12 | 0±0 ** | 14.56±3.86 ** |
| Model Control Group | 12 | 0.52±0.07 | 281.94±43.34 |
| Low-dose Group | 12 | 0.20±0.03 ** | 269.61±34.39 |
| Medium-dose Group | 12 | 0.12±0.02 ** | 191.17±46.21 |
| High-dose Group | 12 | 0.04±0.01 ** | 66.28±17.12 ** |

| | | | |
|---|---|---|---|
| Note: ** indicates p < 0.01 compared with the model control group. | | | |

### 5.4 Cytokines in Supernatants of In Vitro-Stimulated Splenocytes

The results for Th2-type cytokines IL-4, IL-5, IL-10, and IL-13 showed decreases in the medium- and high-dose groups. IL-4 levels in the medium- and high-dose groups were significantly lower than those in the model control group (p < 0.01), while IL-5 and IL-10 levels in the high-dose group were significantly lower than those in the model control group (p < 0.05). These results indicate that treatment with the recombinant protein combination downregulated Th2-type cytokines in model animals, which is closely associated with improvement of allergic responses.

**Table 28 TH2-Type Cytokines in Supernatants of In Vitro-Stimulated Splenocytes (Mean ± SEM)**

| **Group Name** | **Number of Samples (n)** | **Level (pg/mL)** | | | |
|---|---|---|---|---|---|
| | | **IL-4** | **IL-5** | **IL-10** | **IL-13** |
| Negative Control Group | 12 | 0.89±0.65 ** | 25.12±9.71 ** | 238.03±30.96 ** | 323.29±71.83 ** |
| Model Control Group | 12 | 6.76±0.86 | 269.37±39.56 | 1326.03±165.88 | 2280.71±254.09 |
| Low-dose Group | 12 | 4.43±1.07 | 306.32±26.56 | 1799.7±269.20 | 2582.86±83.15 |
| Medium-dose Group | 12 | 2.46±0.39 ** | 261.81±24.58 | 1379.57±96.41 | 2523.47±118.82 |
| High-dose Group | 12 | 2.22±0.39 ** | 211.59±24.28* | 921.46±222.35* | 2186.68±163.43 |

| | | | | | |
|---|---|---|---|---|---|
| Note: * indicates p < 0.05, ** indicates p < 0.01 compared with the model control group. | | | | | |

### 6. Conclusion

The results of the present study demonstrate that, in a mouse model of allergic airway inflammation, the recombinant cat allergen protein combination of the present invention exhibited definite therapeutic efficacy in sublingual immunotherapy. The recombinant protein combination effectively alleviated allergic airway inflammation by downregulating Th2-type cytokine production and IgE levels, and by inhibiting infiltration and activation of inflammatory cells.

## Claims

1. A recombinant Fel d 1 protein, which is a fusion protein of Fel d 1 chain 1 and Fel d 1 chain 2.

2. The recombinant Fel d 1 protein of claim 1, wherein the amino acid sequence of the Fel d 1 chain 1 has at least 67% sequence identity to the sequence set forth in SEQ ID NO: 5; and the amino acid sequence of the Fel d 1 chain 2 has at least 67% sequence identity to the sequence set forth in SEQ ID NO: 6.

3. The recombinant Fel d 1 protein of claim 1, wherein the Fel d 1 chain 1 and Fel d 1 chain 2 are fused directly or fused via a linker peptide; and wherein the Fel d 1 chain 1 and the Fel d 1 chain 2 are located at the N-terminus and the C-terminus of the fusion protein, respectively, or at the C-terminus and the N-terminus of the fusion protein, respectively.

4. The recombinant Fel d 1 protein of claim 1, wherein the amino acid sequence of the Fel d 1 is as set forth in SEQ ID NO: 1 or SEQ ID NO: 2, or wherein the formylmethionine residue at position 1 of the N-terminus of the amino acid sequence set forth in SEQ ID NO: 1 or SEQ ID NO: 2 is absent or is replaced with a methionine residue.

5. A nucleic acid encoding the recombinant Fel d 1 protein of claim 4, wherein the nucleotide sequence thereof is SEQ ID NO: 3 or SEQ ID NO: 4.

6. A Fel d 4 mutant or a derivative thereof, comprising a mutation in a protein sequence of a native Fel d 4 or an isoallergen or variant of the native Fel d 4; wherein the amino acid sequence of the native Fel d 4 is as set forth in SEQ ID NO: 7, and a mutation site is at position 139 of the native Fel d 4 protein sequence; wherein a mutation site of the isoallergen or variant of the native Fel d 4 is at a position corresponding to position 139 of SEQ ID NO: 7; and wherein the mutation is a deletion or a substitution with another amino acid.

7. The Fel d 4 mutant or derivative thereof of claim 6, wherein the mutation site of the Fel d 4 mutant is substituted with one or more amino acids selected from the group consisting of G, A, V, L, I, Y, S, K, R, H, F, W, M, and T.

8. The Fel d 4 mutant or derivative thereof of claim 6, further comprising an additional methionine residue or formylmethionine residue at the N-terminus.

9. A nucleic acid encoding the Fel d 4 mutant or derivative thereof of claim 6.

10. A Fel d 7 mutant or a derivative thereof, comprising a mutation in a protein sequence of a native Fel d 7 or an isoallergen or variant of the native Fel d 7; wherein the amino acid sequence of the native Fel d 7 is as set forth in SEQ ID NO: 10, and a mutation site is at position 101 of the native Fel d 7 protein sequence; wherein a mutation site of the isoallergen or variant of the native Fel d 7 is at a position corresponding to position 101 of SEQ ID NO: 10; and wherein the mutation is a deletion or a substitution with another amino acid.

11. The Fel d 7 mutant or derivative thereof of claim 10, wherein the mutation site of the Fel d 7 mutant is substituted with one or more amino acids selected from the group consisting of G, A, V, L, I, Y, S, K, R, H, F, W, M, and T.

12. The Fel d 7 mutant or derivative thereof of claim 10, further comprising an additional methionine residue or formylmethionine residue at the N-terminus.

13. A nucleic acid encoding the Fel d 7 mutant or derivative thereof of claim 10.

14. A vector comprising the nucleic acid of claim 5, 9, or 13, wherein the vector is a T7 promoter-based expression vector selected from the group consisting of pET32a, pET26b, pET28a, and pDEST14, or a temperature-controlled promoter PL-PR-based expression vector pBV220.

15. An Escherichia coli strain comprising the expression vector of claim 14, wherein:
when the expression vector is based on the PL-PR promoter, the host strain is selected from BL21(DE3), BL21 AI(DE3), Top10, DH5α, JM109, Rosetta(DE3), Rosetta gamiB(DE3), and BL21(DE3)pLysS; or
when the expression vector is based on the T7 promoter, the host strain is selected from BL21(DE3), BL21 AI(DE3), Rosetta(DE3), Rosetta gamiB(DE3), and BL21(DE3)pLys.

16. A method for expressing a recombinant Fel d 1 protein, a Fel d 4 mutant or derivative thereof, or a Fel d 7 mutant or derivative thereof, the method comprising:
A. constructing the vector of claim 14;
B. linearizing the vector of step A, transforming the linearized vector into an Escherichia coli strain, and culturing the strain under suitable conditions; and
C. recovering and purifying the protein.

17. A method for purifying a recombinant Fel d 1 protein, a Fel d 4 mutant or derivative thereof, or a Fel d 7 mutant or derivative thereof, the method comprising:
A. resuspending and disrupting Escherichia coli cells obtained from the culturing in claim 16, and collecting a pellet or a supernatant, wherein the pellet is a crude inclusion body preparation of a target protein;
B. when the inclusion body is collected, subjecting the inclusion body to crude purification, denaturation, and renaturation to obtain a renaturation solution; or, when the supernatant is collected, filtering and concentrating the supernatant with an ultrafiltration membrane to obtain a filtrate; and
C. collecting the renaturation solution or the filtrate, and performing a two-step purification by ion-exchange chromatography and hydrophobic chromatography to obtain a purified target protein.

18. Use of the recombinant Fel d 1 protein of any one of claims 1-4, the Fel d 4 mutant or derivative thereof of any one of claims 6-8, or the Fel d 7 mutant or derivative thereof of any one of claims 10-12 in the manufacture of a medicament for treating a cat allergen-induced allergic disease or in the manufacture of a diagnostic reagent for cat allergy diagnosis.

19. A pharmaceutical composition for treating a cat allergen-induced allergic disease, comprising Fel d 1, Fel d 4, and Fel d 7.

20. The composition of claim 19, wherein:
the Fel d 1 is the recombinant Fel d 1 protein of any one of claims 1-4;
and/or the Fel d 4 is the Fel d 4 mutant or derivative thereof of any one of claims 6-8;
and/or the Fel d 7 is the Fel d 7 mutant or derivative thereof of any one of claims 10-12.
